# EUROPEAN PATENT APPLICATION

(11) **EP 4 252 542 A2**
(43) Date of publication of application: **04.10.2023**
(21) Application number: 23177428.2
(22) Date of filing: 23.08.2019
(51) Int. Cl.: A23K 10/30

(54) **LIVESTOCK FEED ADDITIVE**

(30) Priority: 23.08.2018 EP 18190396
(62) Divisional of application: 19755931.3
(71) Applicant: Delacon Biotechnik GmbH, 4209 Engerwitzdorf (AT)
(72) Inventor: MUELLER, Andreas Stefan, Gramastetten (AT)
(74) Representative: Forresters IP LLP

(57) **Abstract**

A livestock feed additive comprising 1,8 cineole, and at least one of naringin or betalain, and a feed product comprising the same.

## Description

The invention refers to a livestock feed additive and a respective food product, which is supports animals breeding under heat stress.

### BACKGROUND

Zootechnical additives are commonly used to improve the nutritional value of an animal's diet. This category includes, among others, enzymes and certain phytogenics. Phytogenic (plant derived, natural, botanical) feed additives are well-blended compositions of special plant-based raw materials and plant derived and/or mineral-based carriers. For this purpose, essential and/or vegetable oils, as well as a vast range of highly active herbs and spices with special aromatic and appetizing properties may be used.

Phytogenic products are being used as a zootechnical additive, developed to improve the performance of animals, e.g. to improve the health of poultry and/or for profitable poultry production, especially for fattening and egg production. Phytogenic products are considered as natural alternative to the synthetic products that consumers want to avoid, including feed manufacturers, premixers and animal producers.

In the European Union, currently the revision of REGULATION (EC) No 1831/2003 OF THE EUROPEAN PARLIAMENT AND OF THE COUNCIL of 22 September 2003 on additives for use in animal nutrition is in progress. Currently, the legislation defines 5 categories of feed additives mentioned in Article 6:
1. A feed additive shall be allocated to one or more of the following categories, depending on its functions and properties, in accordance with the procedure set out at Articles 7, 8 and 9:
   (a) technological additives: any substance added to feed for a technological purpose;
   (b) sensory additives: any substance, the addition of which to feed improves or changes the organoleptic properties of the feed, or the visual characteristics of the food derived from animals;
   (c) nutritional additives;
   (d) zootechnical additives: any additive used to affect favourably the performance of animals in good health or used to affect favourably the environment;
   (e) coccidiostats and histomonostats.

In the revised regulation it is planned to introduce a new subcategory of (d), dedicated to feed additives improving animal welfare.

Due to the dramatic growth of the world population, currently estimated to reach nearly 10 billion people in 2050, the demand for animal derived food will increase by 40% on the current basis. The largest share of both, the increase in population and the protein demand will take place in newly industrializing countries in Asia, Africa and Latin America. Many of these countries have a hot and humid tropical climate.

Under these climatic conditions intensive rearing of livestock species suffer from heat stress.

For instance, in poultry typical symptoms of heat stress are reflected by the following signs
i. Reduced feed intake and increased water intake
ii. Reduced movement and laying down
iii. Strongly increased breathing rate (up to 250 gasps/min) and gasping to stimulate heat loss by evaporation
iv. Respiratory alkalosis by decrease of partial CO₂-pressure in blood
v. Drastically increased mitochondrial activity (= total metabolic rate) for these compensation mechanisms
vi. At a relative humidity > 50 %, cooling via evaporation is nearly unfeasible, resulting in increased morbidity and mortality

In most recent literature the decrease in performance and increased general morbidity is related to damage to the intestinal barrier integrity in animals under heat stress. The intestinal barrier is composed of several layers providing protection against microbial invasion. The intestinal lumen contains antimicrobial peptides (AMPs), secreted immunoglobulin A (IgA), and commensal bacteria, which inhibit the colonization of pathogens by competitive inhibition and production of e.g. butyrate, which has barrier protective properties. A mucus layer covers the intestinal surface providing a physical barrier. The epithelial layer consists of a single layer of epithelial cells that is sealed by small membrane proteins, referred to as tight junction proteins such as claudins, occludins and zona occludens (ZO1) preventing paracellular passage of noxious molecules and pathogens. This layer also harbors intraepithelial lymphocytes, M cells, mucus producing Goblet cells and bacteriocin-producing Paneth cells. The lamina propria contains a large number of immune cells, both of the innate immune system and the adaptive immune system. A schematic figure of the intestinal barrier and affecting factors is described by König et al. 2016.

It is assumed that under heat stress conditions in particular tight junction proteins are damaged, resulting in a higher exposure of the organism to pathogenic bacteria, their pro-inflammatory cell wall lipopolysaccharides and to other toxic noxa (König et al. 2016).

Pearce et al. (2013 A) describe an increase of serum endotoxin levels and an increase of intestinal heat shock protein expression in growing pigs, which are exposed to heat stress. Under heat stress, at the cellular level, elevated levels of heat shock proteins (HSPs) can be observed. HSPs are molecular chaperons mediating cytoprotection by maintaining proper protein folding, releasing survival signals and preserving cytoskeleton integrity. Pearce et al. (2013 A) indicate that HSPs play a crucial role in maintaining the localization of tight junction (TJ) proteins. In order to compensate for the loss of irreversibly damaged tight junction molecules, an up-regulation of their gene- and protein expression is observed under heat stress. Thus, any damage to the intestinal barrier function is accompanied by a substantial impairment of animal welfare, animal health and animal performance.

Defects in the intestinal barrier are measurable by a reduced Transepithelial Electrical Resistance (TEER) or an increased permeability for large molecules like Fluoresceine Isothiocyanate Dextrane (FITC Dextrane) (Pearce et al. 2013 B).

Citrus fruits, including oranges, grapefruits, lemons, limes, tangerines, and mandarins, belong to the most commonly cultivated fruits. Global production is annually increasing to comply the rising consumer's demands. The citrus-processing industry produces tremendous amounts of wastes. Citrus peel waste accounts for almost 50% of the wet fruit mass. Citrus waste is of high economic value since it abundantly contains various flavonoids, carotenoids, dietary fiber, sugars, polyphenols, essential oils, and ascorbic acid, as well as considerable amounts of some trace elements (Sharma et al. 2017). Citrus fruits contain individual amounts of flavonoids. Whereas the bitter tasting flavonoid Naringin is predominantly contained in pulp and peels of grapefruits and pomelos, hesperidin is the predominant flavonoid of oranges and lemons. Both flavonoids occur in the mentioned citrus fruits both as glycosides (hesperidin and naringin), and as their respective aglycones hesperitin and naringenin (Suntar et al. 2018).

An overview of major flavonoids contained in citrus fruits is provided in the following scheme (scheme 1 from Suntar et al. 2018):

### Flavonoids

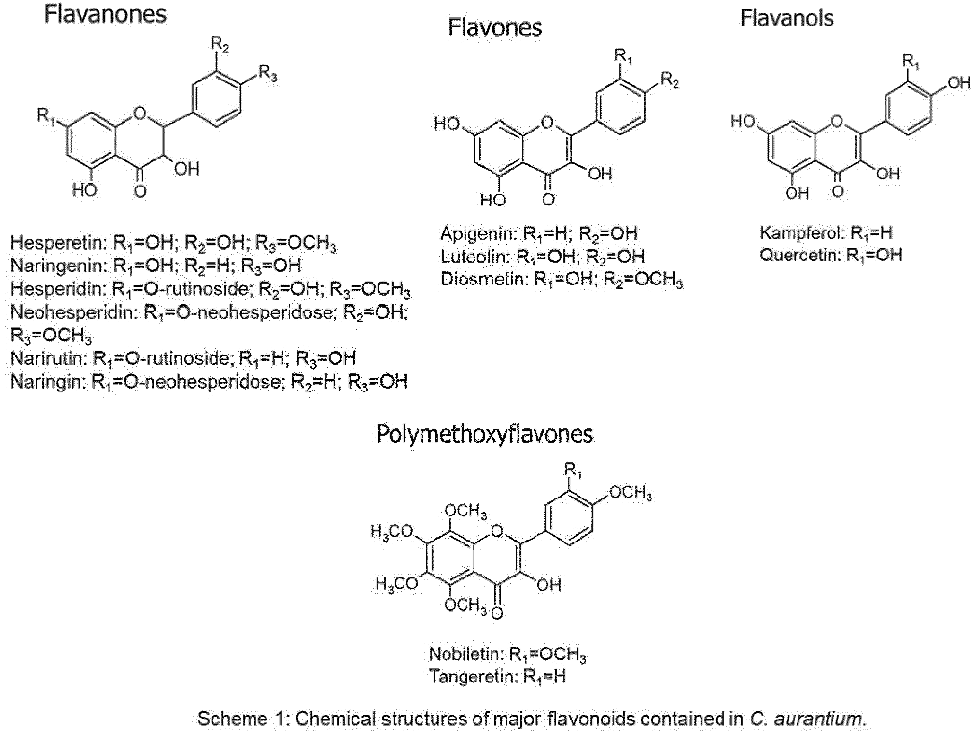

For hesperidin and hesperitin, it could be shown in CaCo2 cells, that the aglycon hesperitin is efficiently absorbed into enterocytes of the small intestine. Subsequently, either free hersperitin or hesperitin conjugates can enter blood circulation. The glycoside hesperidin is predominantly cleaved in the large intestine to rutinose and hesperitin, which can enter the colonocytes and blood circulation as described above. The metabolism and absorption of hesperidin and hesperitin is well-known (Brand et al. 2008).

In general, flavonoids are known to mediate cytoprotection by up-regulating the endogenous antioxidant enzyme system. For both, Hesperidin and Naringin an up-regulation of antioxidant enzymes depending on the transcription factor Nrf2 as master regulator could be shown (Zhu et al. 2017, Chen et al.).

Naringin supported the onset of myocardial repair mechanisms in rats exposed to ischemic stress, via the up-regulation of heat shock proteins (Rani et al. 2013). For chemically purified hesperidin applied at a dietary level of 20 mg/kg diet either alone or in combination with genistein to heat stressed broilers a differential regulation of heat shock protein 70 in breast muscle could be observed (Kamboh et al. 2013). However, the results of this study are in doubt, since the application of hesperidin downregulated the expression of heat shock protein 70. In contrast an up-regulation of heat shock response by specific substances would indicate a support of repair mechanisms.

For both, hesperidin and naringin, it could be demonstrated in CaCo2 cell monolayers, that they support intestinal barrier integrity under unchallenged conditions (Noda et al. 2012). No information is so far available on the protective effects of citrusflavonoids and in particular naringin on the protection of intestinal barrier function under heat stress conditions.

The Lamiaceae family, one of the most important herbal families, incorporates a wide variety of plants with biological and medical applications. The most known members of this family are a variety of aromatic spices like thyme, mint, oregano, basil, sage, savory, rosemary, self-heal, hyssop, lemon balm, and some others with more limited use.

*Origanum Genus. Origanum* is a genus of herbaceous perennials and subshrubs in the Lamiaceae family, native to Europe, North Africa, and also in temperate zones of Asia. A few species are also naturalised in North America and other regions. The plants have strongly aromatic leaves and abundant tubular flowers with long-lasting coloured bracts. The genus includes *Origanum vulgare* L. or common marjoram and *Origanum majorana* L. or sweet marjoram.

*Origanum vulgare. O. vulgare* is an aromatic, woody based perennial plant, native to the stony slopes and rocky mountain regions in the Mediterranean area (Portugal and Andalusia), Europe (including the British Isles), and south and central Asia. The major compounds (terpenes) contained in *Origanum* oil are carvacrol (up to 70%), terpinen-4-ol (26%), cis-sabinene (13.3%), o-cymene (9.3%), γ-terpinen (5.8%), trans-sabinene (5.7%), *p*-menth-1-en-8-ol (5.1%), b-thujene (4.9%), and *α*-terpinen (3.5%).

In traditional folk medicine, *Origanum* was used to treat several illnesses. Beneficial effects reported, include anti-spasmodic, antimicrobial and digestive-enhancing properties.

*Thymus Genus. The Thymus* genus, as part of the Lamiaceae family, consists of over 350 species of aromatic plants with evergreen leaves. Geographically, these plants extend to Asia, North Africa, and Europe. Although more than one species is cultivated for culinary or ornamental use, the most extensively studied species is *Thymus vulgaris.* Used for thousands of years in traditional medicine, *Thymus* species unroll a wide spectrum of therapeutic properties, including antimicrobial, anti-inflammatory and even anti-cancerogenic effects by its apigenin content.

*Thymus vulgaris.* GC-MS and GC-FID analyses revealed that the main active components in one type of *Thymus vulgaris* L. essential oil are thymol (41.0%), geraniol (26.4%), thujanol (42.2% cis-sabinene hydrate and 7.3% *trans*-sabinene hydrate), and linalool (72.5%). Others also contain borneol and carvacrol. The chemotype of thyme is determined based on oil composition. Geographical provenience and weather influence the chemotype and composition, which has been demonstrated in a study comparing essential oils from two regions of France (linalool chemotype with 76.2% linalool and thymol chemotype with 47.1% thymol) and two regions of Serbia (geraniol chemotype with 59.8% geraniol and sabinene hydrate chemotype with 30.8% cis-sabinene hydrate). Thymol (2-isopropyl-5-methylphenol) and 5-eugenol (4-allyl-2-methoxyphenol) are terpenoids of *T. vulgaris* which have been shown to unfold anesthethic effects. Moreover, thymol has been shown to inhibit vitamin K synthesis leading to its potential use as anticoagulant. *T. vulgaris* also has a spasmolytic, antimicrobial, anti-inflammatory, immunomodulatory, and antioxidant characteristics, these effects being attributed to the thymol contained in the volatile thyme oil. Numerous studies have confirmed the beneficial effects of *T. vulgaris* on respiratory pathologies by its spasmolytic and mucolytic features. Other studies have indicated its promising potential in the treatment of gastrointestinal pathologies in animal models without exerting any toxic potential.

*Rosmarinus Genus.* In the Lamiaceae family *Rosmarinus* is a genus of woody, perennial herbs with fragrant evergreen needle-like leaves native to the Mediterranean Basin.

*Rosmarinus officinalis. Rosmarinus officinalis* L., commonly called rosemary, is a Mediterranean shrubby herb and widely spread in European, American, and Asian countries. It is a common spice used worldwide for culinary, medicinal, and commercial purposes in fragrance and food industry. The leaves of rosemary (fresh or dried) are used for their characteristic aroma in cooking or consumed in small amounts as herbal tea, while rosemary extracts are used because of their antioxidant properties to improve the shelf life of perishable foods. Recently, rosemary extracts (E392) have been approved as a safe and effective natural antioxidant for food preservation by the European Union. Phytochemical studies have revealed that leaves contain 0.5% to 2.5% volatile oil. The major components of rosemary oil include monoterpene hydrocarbons (alpha and beta pinene), camphene, limonene, camphor (10% to 20%), borneol, cineole, linalool, and verbinol. Rosemary contains a widespread variety of volatile and aromatic components. Flavonoids in the plant include diosmetin, diosmin, genkwanin, luteolin, hispidulin, and apigenin. Additionally, terpenoid components from rosemary comprise the triterpenes oleanolic and ursolic acid and the diterpene carnosol. Phenols in rosemary include caffeic,- chlorogenic,- labiatic,- neochlorogenic - and rosmarinic acid. Rosemary contains high amounts of salicylates. Recent pharmacological studies have indicated that rosemary and its constituents, especially caffeic acid derivatives such as rosmarinic acid, have various traditional uses in ethnomedicine including analgesic, anti-inflammatory, anti-carcinogenic, anti-rheumatic, spasmolytic, anti-hepatotoxic, anti-atherosclerotic, carminative, and choleretic effects (Uritu et al. 2018).

Hosseini et al. (2018) reported that a mixture of cinnamaldehyde and thymol showed an indifferent response in broilers with social stress (overcrowding stress). In this study, a mixture of thyme oil with cinnamaldehyde has been used. Another study in broilers reported beneficial effects of thyme oil on intestinal integrity (Placha et al. 2014). Positive effects of thyme on general resilience of animals to stress conditions may derive from its potential to induce endogenous antioxidant response via the Nrf2 pathway (Kluth et al. 2007, FangFang et al. 2018). A similar situation exists also for oregano. Zou et al. (2016, (B)) reported that in pigs the essential oil from oregano contributed to an improvement of intestinal morphology and tight junction protein expression, linked to changes in intestinal microflora. Both in vivo and in vitro, carvacrol, the main terpene of oregano oil, could be demonstrated to support heat shock protein induction as one trigger of T-cell response (Wieten et al. 2010). As similarly reported for thyme and thymol part of the antioxidant, effects of oregano may be triggered via induction of the Nrf2 pathway (Zou et al. 2016 (A)).

For rosemary only one study exists in current literature in context with heat stress. Türk et al. (2016) have reported that feeding rosemary oil in very high, but uneconomic concentrations of 125 and 250 g/t of complete feed reduced lipid peroxidation in the testes of quails, thereby reducing reproductive failures.

In animal nutrition commonly practiced strategies to ameliorate adverse effects of heat stress on animal health and performance include the use of:
- Antioxidant vitamins, in particular vitamin C
- Buffer substances and electrolytes, to compensate losses and alkalosis
- Synthetic Betaine (N,N,N-Trimethylammonioacetate), a methyl donor supporting in particular the vitamin metabolism of vitamin B12 and folic acid, as well as methionine metabolism.

Betaine is dosed routinely at levels of 0,5 to 1,0 kg/t of feed.

The use of betaine in the poultry industry to cope with the heat stress problem is reviewed by Saeed et al. (2017). Further studies need to be conducted at the genetic and molecular basis to elucidate the mechanism behind the betaine as a natural anti-heat agent to decrease the heat stress problem in the poultry industry.

Betalains are a class of red and yellow indole-derived pigments found in plants of the Caryophyllales, like barbary, where they replace anthocyanin pigments. Betalains also occur in some higher order fungi like *Tricholomopsis rutilans.* They are most often noticeable in the petals of flowers, but may color the fruits, leaves, stems, and roots of plants that contain them. They include pigments such as those found in beets, in particular of beet root. The name "betalain" comes from the Latin name of the common beet (Beta vulgaris), from which betalains were first extracted. The deep red color of beets, bougainvillea, amaranth, and many cactuses results from the presence of betalain pigments. The particular shades of red to purple are distinctive and unlike that of anthocyanin pigments found in most plants.

There are two categories of betalains:
1. Betacyanins include the reddish to violet betalain pigments. Among the betacyanins present in plants include betanin, isobetanin, probetanin, and neobetanin.
2. Betaxanthins are those betalain pigments which appear yellow to orange. Among the betaxanthins present in plants include vulgaxanthin, miraxanthin, portulaxanthin, and indicaxanthin.

Betanin is a betalain, also called beetroot red, because it may be extracted from red beet roots.

Betalains have been shown to exert antioxidants either directly by comparison with Trolox^{®} antioxidant potential or by induction of antioxidant and detoxifying enzymes via the nrf2 pathway (Lee et al. 2005, Esatbeyouglu et al. 2015).

AU2005229753A1 discloses botanical compositions for optimal health and prevention of illness through the supply of nutrients, thereby improving a great variety of illnesses. A series of different chemicals are disclosed for certain purposes. Amongst the antifatigue chemicals,1 ,8-cineole is mentioned. The list of disclosed anti-myorelaxant chemicals includes *inter alia* 1,8-cineole and thymol. The list of anti-inflammatory chemicals includes *inter alia* 1,8-cineole and naringin.

US4759932 discloses a method of reducing heat stress in animals by adding zeolite to the feed.

US2008032021A1 discloses a poultry feed additive comprising an essential oil derived from at least one of a herb and a spice and containing thymol and carvacrol as its main ingredients, and at least one organic acid derived from at least one citric, fumaric, fulvic and humic acid.

Maria Miguel provides a review of the betalains present in some species of the Amaranthaceae family (Maria Miguel 2018).

WO2017037157A1 discloses a poultry feed additive composition which is a flowable mixture of phytogenic compounds comprising a microencapsulated essential thyme oil, and saponin contained in particulate dried quillaja bark powder, to enhance digestibility and performance for fattening or laying or breeding, to point-of-lay.

There is a need for improved livestock feeds which allows improved breeding under heat stress, contributing to animal welfare.

### SUMMARY OF THE INVENTION

It is the object of the present invention to provide a new livestock feed additive or feed product with specific components to improve the animal's resistance to heat stress while breeding.

The object is solved by the subject matter as claimed.

According to the invention there is provided a livestock feed additive comprising 1,8 cineole, and at least one of naringin or betalain.

Specifically, the livestock feed additive comprises at least 1,5% 1,8 cineole (w/w) and at least one of naringin or betalain.

Specifically, the feed additive comprises at least 2% 1,8 cineole (w/w), preferably up to 5.5% or up to 6% (w/w).

Specifically, the feed additive comprises at least 3% naringin (w/w), preferably up to 6% (w/w), and/or at least 0,1% betalain (w/w), preferably up to 3% (w/w).

Specifically, the feed additive comprises
a) 1,8 cineole and naringin at a ratio of at least 0.2:1 (w/w); and/or
b) 1,8 cineole and betalain at a ratio of at least 4:1 (w/w).

Specifically, the ratio of 1,8 cineole per naringin is at least any one of 0.125:1, 0.2:1, 0.25:1, 0.5:1, 0.75:1, 1:1, 1.25:1, preferably up to 1.5:1 (w/w). A preferred ratio of 1,8 cineole per naringin is in the range of 0.5:1 - 1.5:1 (w/w) or 0.5:1 - 1:1 (w/w).

Specifically, the ratio of 1,8 cineole per betalain is at least any one of 4:1, 5:1, 6:1; 7:1, 8:1, 9:1, 10:1, 11:1, 15:1, 20:1, 22:1, 25:1, 30:1, 33:1, 35:1, 40:1, 44:1, 45:1, 50:1, 55:1, 60:1, preferably up to 64:1 (w/w). A preferred ratio of 1,8 cineole per betalain is in the range of 10:1 - 30:1 (w/w).

Specifically, the 1,8 cineole is provided as a 1,8 cineole component of the feed additive.

Specifically, the naringin is provided as a naringin component of the feed additive.

Specifically, the betalain is provided as a betalain component of the feed additive.

Specifically, the feed additive comprises components comprising 1,8 cineole, and at least one of a component comprising naringin or a component comprising betalain, which components are each phytogenic.

Specifically, the 1,8 cineole is provided as a pure or purified compound, or as essential oil of aromatic plants comprising 1,8 cineole, such as a natural or native essential oil of plants of as of the Lamiaceae family e.g., of aromatic spices like thyme, mint, oregano, basil, sage, savory, rosemary, self-heal, hyssop, lemon balm, but also of camphor laurel, bay leaves, tea tree, sweet basil wormwood, rosemary, common sage, or *Cannabis sativa.* According to specific embodiments, nature identic essential oils of any of the above mentioned plant species, or artificial compositions comprising purified or pure 1,8 cineole are used as 1,8 cineole sources.

Specifically, the 1,8 cineole is comprised in an essential oil, or non-essential oil, preferably of lamiaceae plants, preferably of rosemarinus genus. Specifically, the essential oil is essential rosemary oil.

According to a specific embodiment, the essential oil component is not microencapsulated. However, according to an alternative embodiment, microencapsulated essential oils can serve as the 1,8 cineole source. Specifically, the essential oil comprising 1,8 cineole is microencapsulated.

Specifically, the naringin is provided as a pure or purified compound, or as flavonoid-containing plant product, such as citrusflavonoids containing products originating from citrus plants.

Specifically, the naringin is comprised in citrus plant material, preferably of orange or grapefruit, in particular of citrus fruit peels or pomace.

Specifically, the betalain is provided as a a pure or purified compound, or as betalain pigments-containing plant product, such as originating from beet. Specifically, the betalain is any one or more of betanin, isobetanin, probetanin, and neobetanin. Specifically, the betalain is of red beet roots.

Specifically, the betalain is comprised in biological material of plants of the Caryophyllales order, preferably of the Amaranthaceae family, preferably of beta vulgaris, beet root, or barbary, or of fungi (e.g., *Tricholomopsis rutilans*).

Typically, the feed additive comprises the components 1,8 cineole, and naringin and/or betalain in a standardized amount and in the concentrated form.

Specifically, the feed additive comprises or consists of is a flowable mixture of phytogenic compounds, and optionally further comprising auxiliary agents or excipients.

Specifically, the feed additive comprises or consists of a flowable mixture comprising phytogenic compounds. Specifically, such phytogenic compounds are selected from the group consisting of essential oils, carbohydrates, dried herbs, spices. Specificlaly, the knowable mixture further comprises any one or more of bulking agents, anti-caking agents, or further excipients.

Specifically, the feed additive further comprises one or more of essential oils, which are optionally microencapsulated, or in the free form, dried herbs, spices, carbohydrates, bulking or anti-caking agents, or further excipients.

Specifically, the essential oil comprising 1,8 cineole and optionally further essential oils described herein, are produced by spray-drying an oil in water (o/w) emulsion.

Typically, the essential oil is homogeneously mixed with dry substances to produce the feed additive which is a dry, flowable oil in solid (o/s) mixture or dispersion that can be stored at room temperature over a prolonged period of time.

For example, the feed additive described herein can be pelleted as such, or together with further nutritional feed substances to produce a pelleted feed product without significant losses of the essential oil during production and even after long-term storage. Specifically, the feed additive or feed product is provided as storage stable, powder, or pelletable or pelleted preparation, with a stability of at least 18 months at room temperature, or a temperature of up to 25°C. Upon prolonged storage, at least 65% of the essential oil can still remain in the feed product, preferably at least 70%,

The feed additive described herein preferably contains 20-60% (w/w) dried herbs. Amongst the preferred dried herbs there are powders of angelica root, anise, apple, artichoke, balm, barberry, basil, birch leaves, bitter orange, black currant, blackberry, blueberry, calamus, cassia, cedar, chamomile, chestnut, cimicifuga, cinnamon, citronella, clover, coltsfoot, common centaury, common nettle, dandelion, dill, elder flower, eucalyptus, fennel, gentian, ginger, ginko, grape seed, grape seeds, grapefruit, greater celandine, green tea, hawthorne, hop, horseradish, horsetail, hyssop, ipecac, lemon, lemon balm, lemongrass, licorice, lovage, maca, marigold, marjoram, marshmallow, mint, mistletoe, mugwort, muira puama, oak wood, olive leaves, orange, oregano, parsley, passiflora, peppermint, pine needle, purple coneflower, psyllium, quassia, quebracho, quillaja, raspberry leaves, ribwort, rose, rosemary, rue, rose hips, saflor, sage, sandalwood, savory, Siberian ginseng, soapwort, st. johnswort, star anise, taiga root, tea, thyme, tormentil, valerian root, wormwood, yarrow, or yohimbe bark.

The feed additive described herein may further include dried spices, e.g. 1-15% (w/w), like allspice, camphor, caraway, cardamom, cayenne pepper, clove, coriander, cumin, curcuma, fenugreek, garlic, juniper berry, nutmeg, onion, paprika, pepper, or turmeric

Further excipients may be used which are bulking and anti-caking agents, e.g. in an amount ranging 20-60% (w/w), organic sources like wheat bran, rice bran or other grain brans, modified starch, lactose or dextrose and anorganic sources like silicon dioxide, limestone orbentonite.

Specifically, the feed additive comprises a phytogenic saponin, such as contained in particulate dried quillaja bark powder, preferably containing saponin in an amount ranging from 3 to 10 % (w/w).

Specifically, the feed additive further comprises at least one essential oil comprising carvacrol and/or thymol.

According to a specific embodiment, the essential oil component comprising 1,8 cineole is also comprising carvacrol and/or thymol. Thus, a mixture of 1,8 cineole, carvacrol and/or thymol can be used.

According to a further specific embodiment, a mixture of essential oils is used, wherein 1,8 cineole is provided in an essential oil component, and any one or both of carvacrol or thymol are provided in an additional essential oil component.

Preferably, the essential oil comprising carvacrol and/or thymol is a plant extract. Suitable sources of carvacrol and/or thymol are aromatic plant species, such as thyme or oregano. Specifically, the feed additive comprises at least 0,05% or at least 0,1% carvacrol (w/w) and/or at least 0,05% or at least 0,1% thymol (w/w).

Specifically, essential oils are comprised in the feed additive described herein in an amount ranging from 4% to 16% (w/w) in total.

Specifically, essential oils as used in the feed additive described herein are provided as essential oil extract of plant material.

Specifically, the feed additive is provided as a storage stable, pelletable or pelleted dry preparation, with a stability of at least 18 months at room temperature.

Specifically, the feed additive is used to prepare a feed product including said feed additive e.g., by mixing with nutrients and/or excipients. Specifically, the feed additive is provided in a preparation for use in preparing a feed product for poultry, swine or ruminants.

The invention further provides a feed product comprising the feed additive described herein. Specifically, the feed product comprises 1,8 cineole at a concentration of at least 5 mg/kg, or at least 6 mg/kg. Preferable upper limits of 1,8 cineole are any one of 20, 12, or 10 mg/kg.

Specifically, the feed product comprises 1,8 cineole at a concentration in the range of 5-20 mg/kg, or 6-18 mg/kg, or 6-12 mg/kg, in particular about (+/-2 mg) 10 mg/kg.

Specifically, the feed product comprises naringin at a concentration of at least 5 or 10 mg/kg, or at least 11 or 12 mg/kg. Preferable upper limits of naringin are any one of 30, 24, or 18 mg/kg.

Specifically, the feed product comprises naringin at a concentration in the range of 5-25 mg/kg, or 12-30 mg/kg, in particular about (+/-2 mg) 15 mg/kg.

Specifically, the feed product comprises betalain at a concentration of at least 0.2 or 0,3 mg/kg, or at least 0,4 or 0,5 mg/kg. Preferable upper limits of betalain are any one of 2.4, 1.2, or 0.6 mg/kg.

Specifically, the feed product comprises betalain at a concentration in the range of 0.2-2.0 mg/kg, or 0.5-1.8 mg/kg, in particular about (+/-0.21 mg) 1.7 mg/kg.

Specifically, the feed product is antibiotic-free.

According to a specific embodiment, the feed product comprises the feed additive at a dose of at least any one of 100, 150, 200, 250, 300, 400, or 500 g/t (gram additive per ton feed product), preferably within the range of 100-1000 g/t, or 200-500 g/t, e.g., up to 750 mg/kg, or up to 400 mg/kg of the feed product, or in particular 400 g/t or 500 g/t.

Specifically, the feed product is provided as a premixture to produce complete feed (used as complete diet), or a complete feed, not requiring further nutrients. Specifically, a preferred dosage (concentration) of the feed additive is ranging from 200 to 500 mg/kg, or ranging from 200 to 400 mg/kg of complete feed.

For example, the feed additive is admixed to a feed material e.g., bruised grain, by-products from the alcohol production based on grains, oil seeds like rapeseed, linseed, soybean and by-products from the oil production like soybean meal, rapeseed meal, rapeseed cake, linseed meal, linseed cake or by-product from the fish and meat processing industry like fish meal, meat meal, meat and bone meal, feather meal, blood meal and hydrolyzed animal derived proteins and mineral premixtures, and preferably pelleted.

Specifically, the feed product is provided in the pelleted form.

The feed additive or feed product described herein is preferably pelleted by a process of compressing or molding the material into the shape of a pellet.

According to a specific aspect, the feed additive or the feed product described herein are used for breeding livestock.

Specifically, the feed additive or the feed product described herein is used for breeding livestock which are at risk of or suffering from heat stress.

According to a specific aspect, the feed additive or feed product described herein are used for improved feed conversion in livestock. Specifically, the improved feed conversion is determined by an increased nutrient digestibility or a decreased feed conversion rate *e.g*., as determined by the respective *in vitro* or *in vivo* models.

Specifically, the feed additive or feed product described herein is used as digestibility and performance enhancer *e.g*., according to the definition of Regulation (EC) 1831/2003, in poultry for fattening or laying or breeding, to point-of-lay.

According to a specific aspect, the invention provides for a method of breeding livestock at risk of or suffering from heat stress comprising feeding the feed product described herein.

According to a specific aspect, there is further provided a method of feeding a livestock animal with a feed product described herein, wherein said feed results in an improvement in one or more of said animal's biophysical characteristics when compared to the equivalent use of a feed product that does not comprise the feed additive described herein, in particular wherein the animal is exposed to heat stress over a period of at least 7 or at least 14 days (consecutive days or in total during the breeding time). Heat stress is herein understood as a condition resulting from elevated breeding room temperatures, typically wherein the temperature is 28 to 35°C during daytime, and 22 to 28°C during nighttime.

Specifically, said feed results in an improvement of the feed conversion efficiency. Typically, said improvement is an increase in (average) daily weight gain, preferably of at least 1.0 % or at least 2.0%, over a period of 35 days, when the composition is dosed at an amount of at least 100 or 200 mg per kg feed product.

In particular, said feed results in a higher growth performing efficacy than the commercially used betaine.

Specifically, said improvement results in a decrease in the feed conversion ratio, preferably of at least 1.5 % or at least 2%, over a period of at least 35 days, when the composition is dosed at an amount of at least 100 or 200 mg per kg feed product.

Specifically, said improvement results in an increased protein digestibility, preferably of at least 1 % over a period of at least 35 days, when the composition is dosed at an amount of at least 100 or 200 mg per kg feed product. An exemplary dose range for the additive is 200-400 mg/kg feed, or 200-500 mg/kg feed.

The very specific mode of action of the 1,8 cineole component in combination with a naringin component or a betalain component of the feed additive described herein allows for the formulation of phytogenic feed additives, feed products and respective feeds targeting the maintenance of intestinal barrier function, and with it animal welfare, animal health and animal performance.

In particular, the feed additive is a phytogenic poultry feed additive contributing to the maintenance of animal welfare in poultry, such as e.g., chicken (broilers or layers), or turkey), pigs, or ruminants.

Specifically, the feed additive or feed product described herein is indicated for breeding under heat stress conditions, aiming at:
▪ Improvement of performance, and/or
▪ Improvement of heat shock response, and/or
▪ Improvement of intestinal barrier integrity.

Therefore, the present invention is based on the surprising findings on the effects of essential oils of lamiaceae, such as a lamiaceae oil mixture (e.g., a mixture of oils of one or more plant or species sources) in combination with naringin or betalain on the regulation of heats shock proteins in intestinal cells, the heat shock response and preservation of intestinal barrier function in animals which are under exposure to heat stress.

### FIGURES

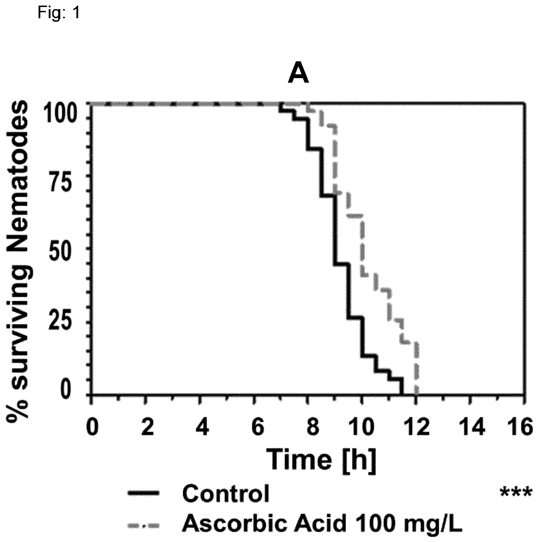

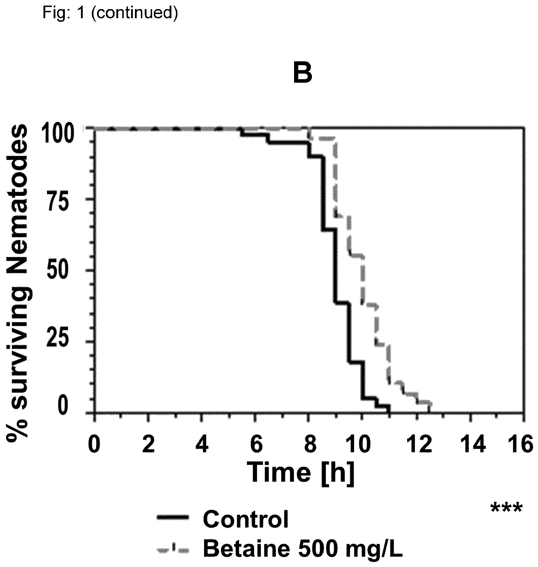

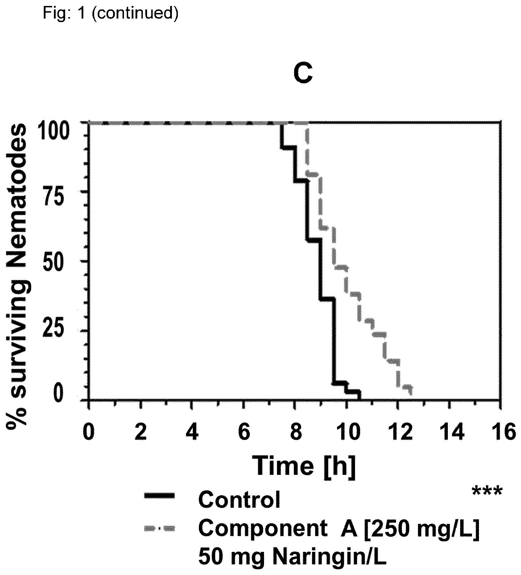

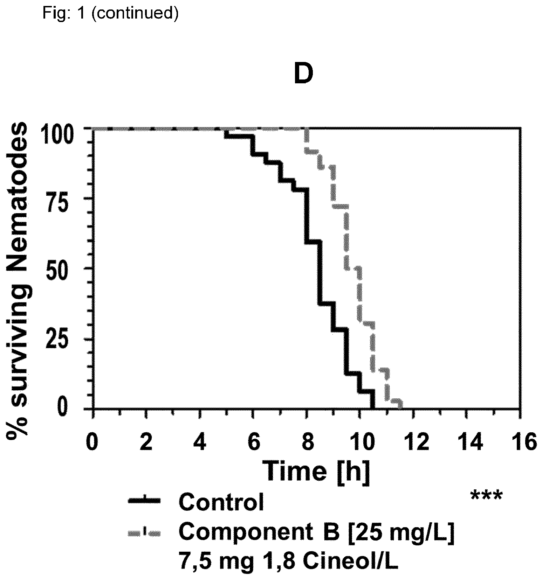

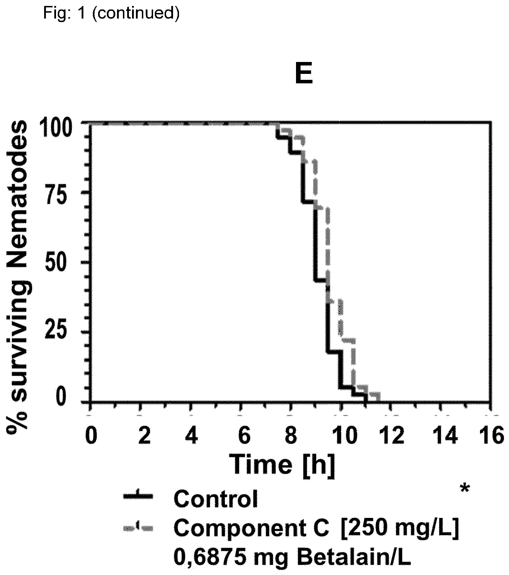

Fig. 1: A, B, C, D, E: Trial I: Life span extension in *C. elegans* under heat stress by reference substances (Ascorbic acid, Betaine) and compounds "A", "B", and "C".

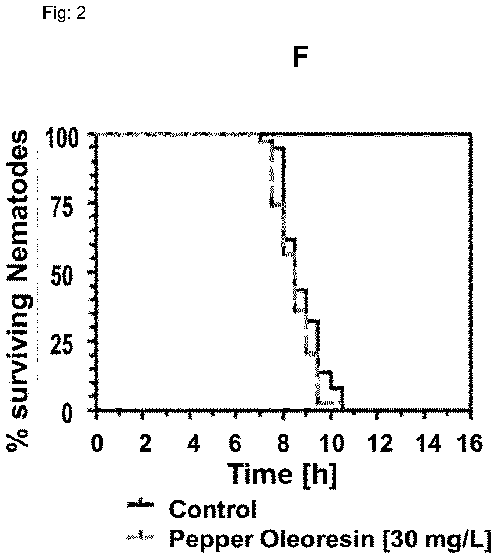

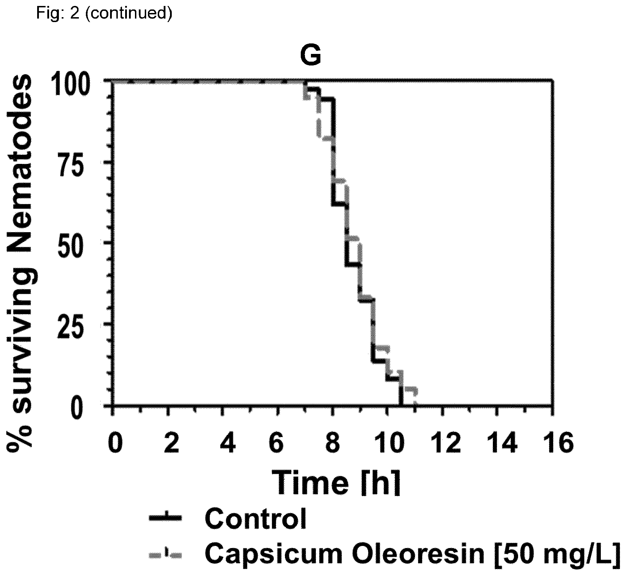

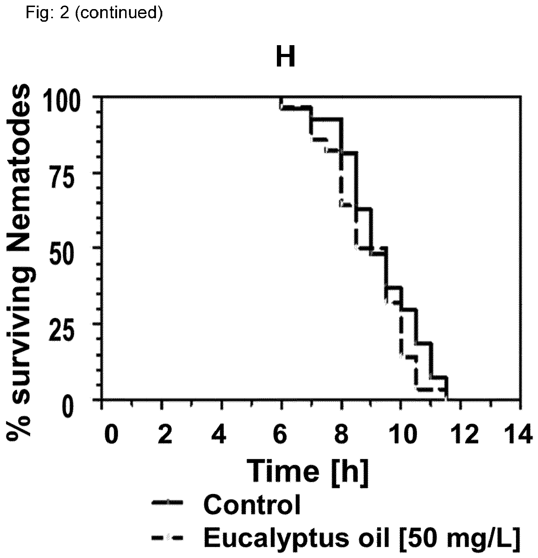

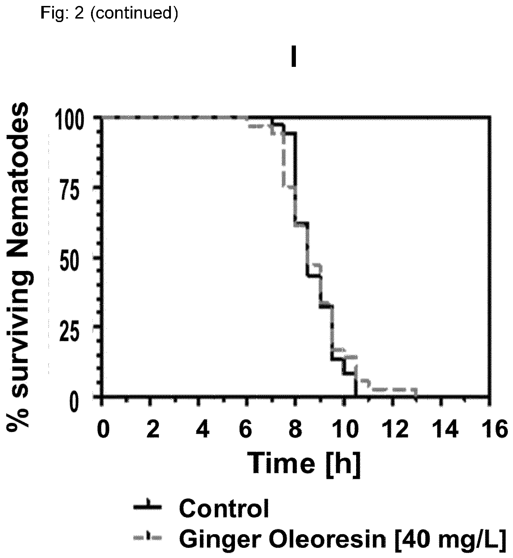

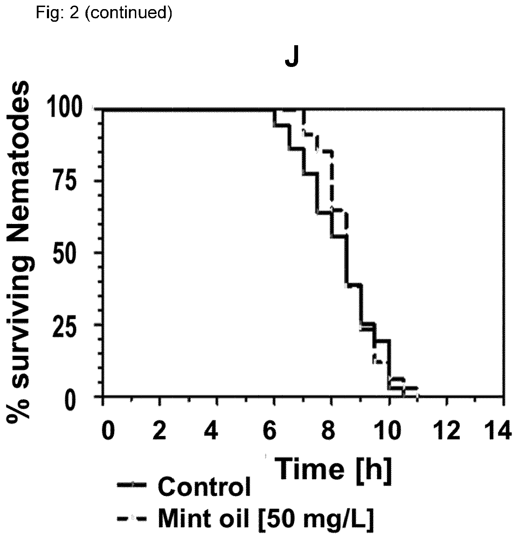

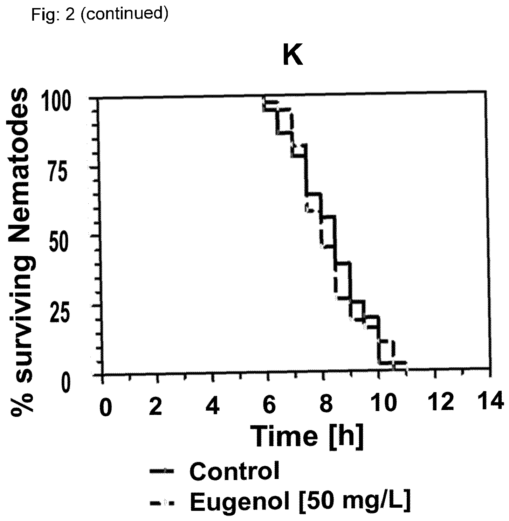

Fig. 2: F, G, H, I, J, K: Trial I: Comparative example: Life span extension in C. *elegans* under heat stress by further substances, frequently recommended to lower negative consequences of heat stress (Piperine, Capsaicin, Gingerol, Eucalyptus oil, Mint oil, Eugenol).

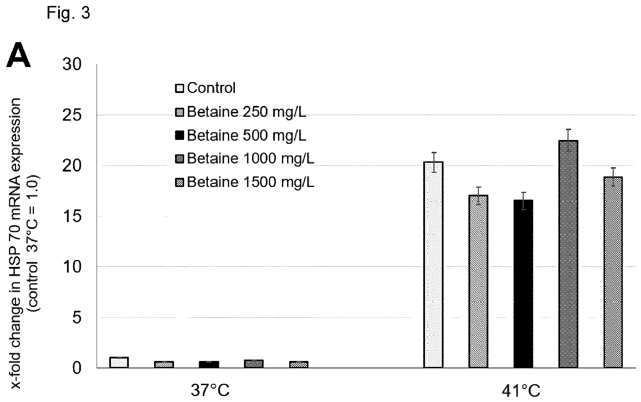

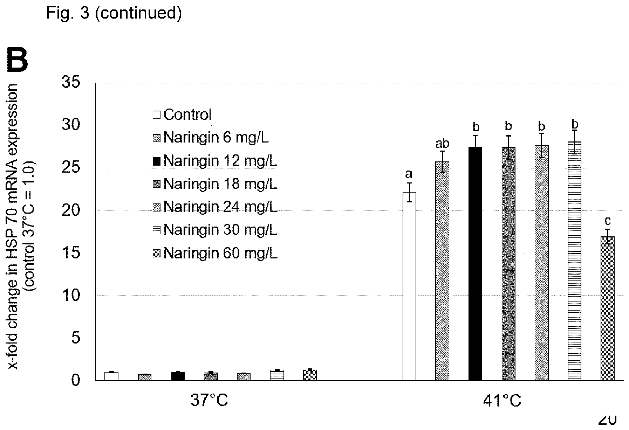

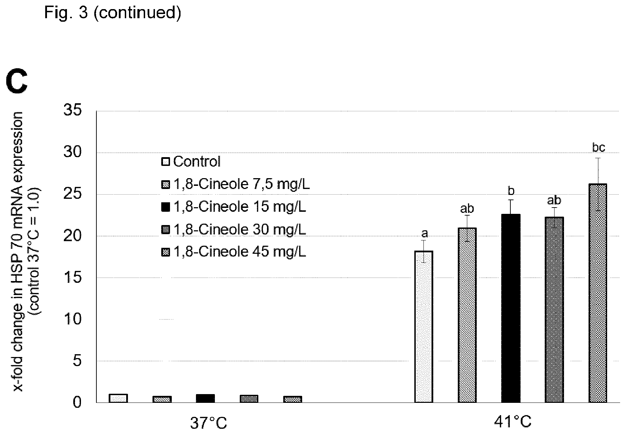

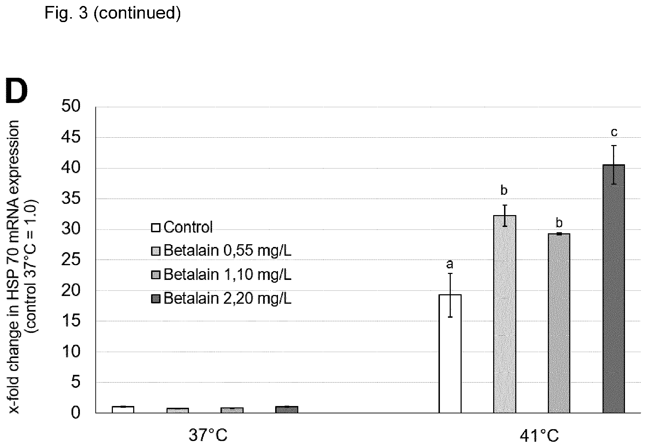

Fig. 3: Trial II: Dose-dependent induction of heat shock response in CaCo2 cells under heat stress by A) Betaine (reference substance), B), C), D) compounds "A", "B", "C"; and E) the combination of compounds "B" and "C" vs. compounds "B" and "C" alone.

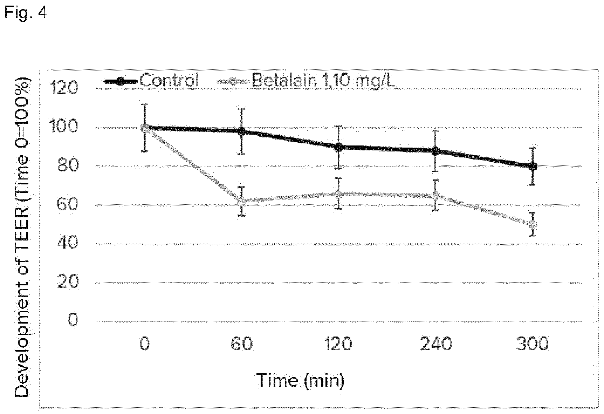

Fig. 4: Trial II: Effect of betalain (compound "C") at 1,10 mg/L on TEER development of transwell CaCo2 cell cultures exposed to heat stress at 42°C.

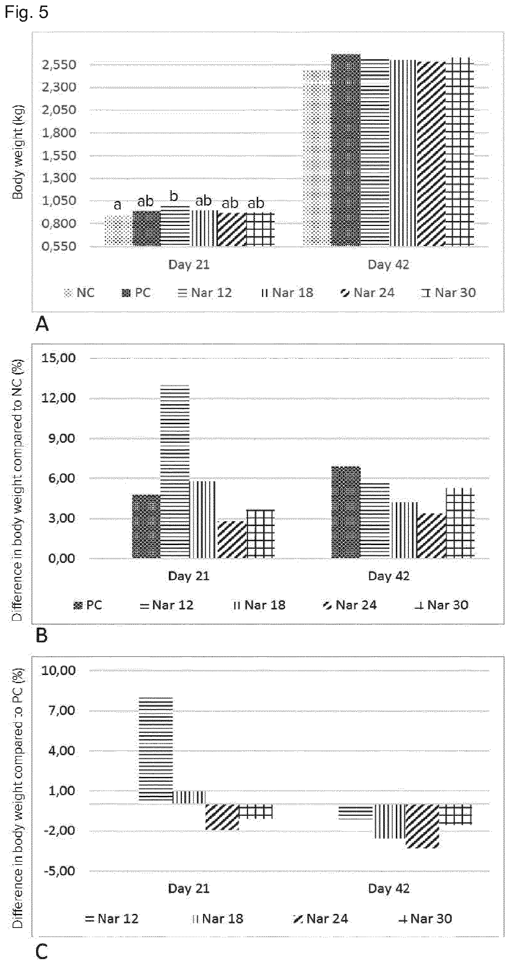

Fig. 5: Trial IV: Effect of betaine (reference substance) and compound "A" on body weight at days 21 and 42 in broilers exposed to heat stress from day 22 to day 42 - A) absolute body weight; B) relative difference of betaine and compound A to the negative control; C) relative difference of compound A to betaine; *a,b,c: different small letters indicate significant differences between means.

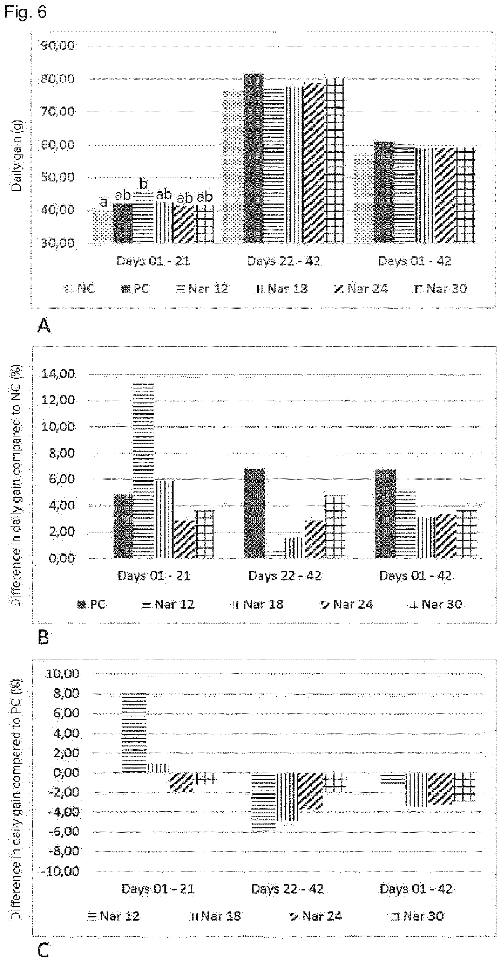

Fig. 6: Trial IV: Effect of betaine (reference substance) and compound "A" on daily weight gain in the periods 0-21d, 22-42d and 0-42d in broilers exposed to heat stress from day 22 to day 42 - A) absolute daily weight gain; B) relative difference of betaine and compound "A" to negative control; C) relative difference of compound "A" compared to betaine; *a,b,c: different small letters indicate significant differences between means.

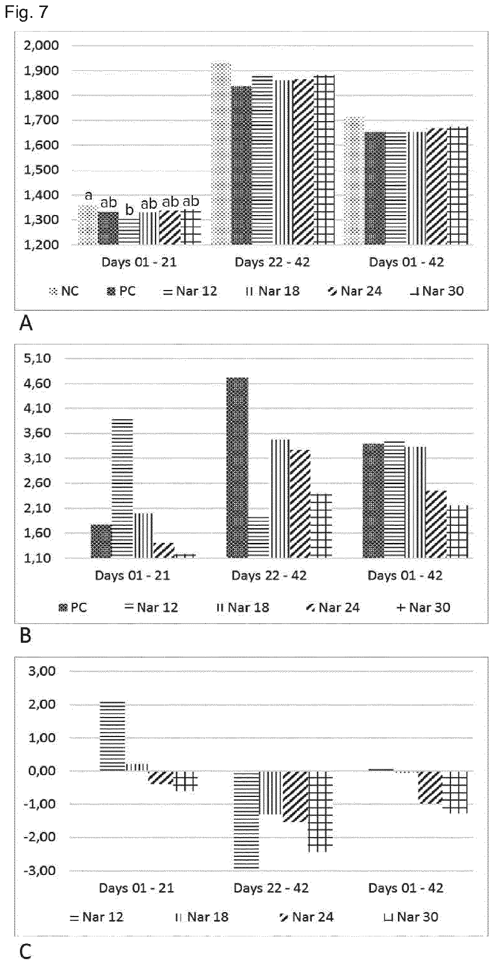

Fig. 7: Trial IV: Effect of betaine (reference substance) and compound "A" on FCR in the periods 0-21d, 22-42d and 0-42d in broilers exposed to heat stress from day 22 to day 42 - A) absolute FCR; B) relative to the negative control; C) relative to the positive control betain; *a,b,c: different small letters indicate significant differences between means

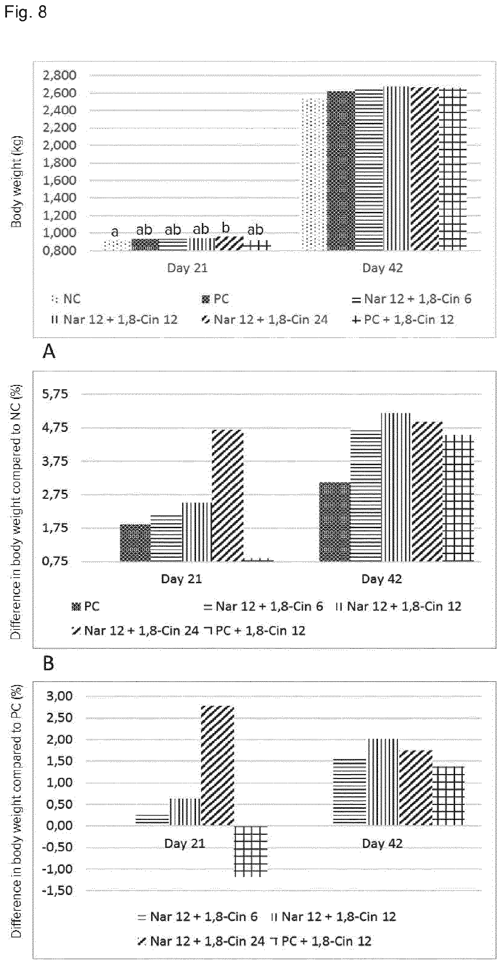

Fig. 8: Trial V: Effect of betaine (reference substance) and combinations of compound "A" with different concentrations of compound "B" on body weight at days 21 and 42 in broilers exposed to heat stress from day 22 to day 42 - A) absolute body weight; B) relative difference of betaine and combinations of compound "A" and "B" to the negative control; C) relative difference of combinations of compound "A" and "B" to betaine; *a,b,c: different small letters indicate significant differences between means.

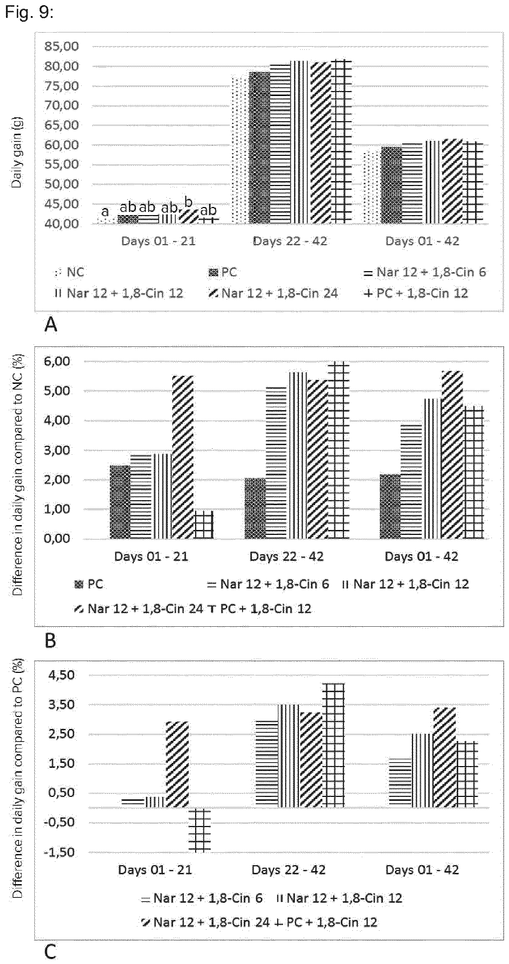

Fig. 9: Trial V: Effect of betaine (reference substance) and combinations of compound "A" with different concentrations of compound "B" on daily weight gain in the periods 0-21d, 22-42d and 0-42d in broilers exposed to heat stress from day 22 to day 42 - A) absolute daily weight gain; B) relative difference of betaine and combinations of compound "A" and "B" to the negative control; C) relative difference of combinations of compound "A" and "B" to betain; *a,b,c: different small letters indicate significant differences between means.

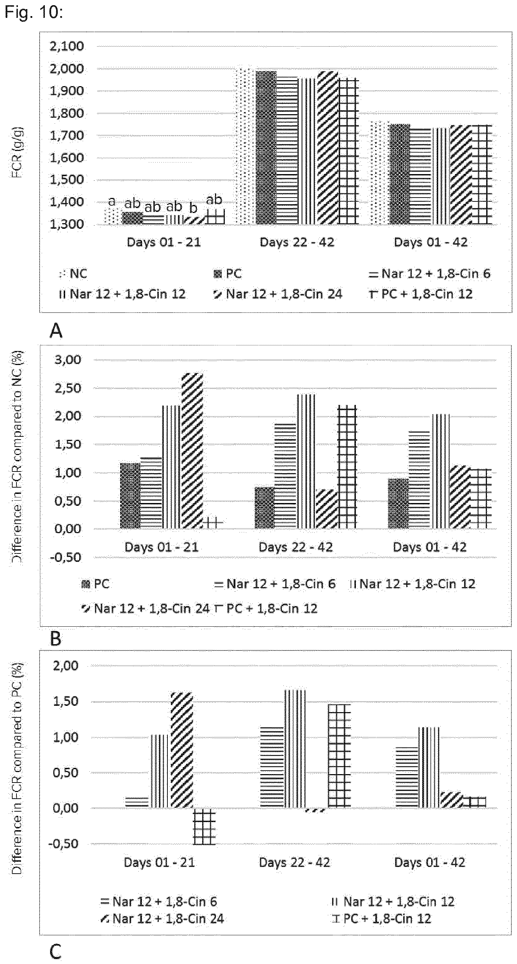

Fig. 10: Trial V: Effect of betaine (reference substance) and combinations of compound "A" with different concentrations of compound "B" on FCR in the periods 0-21d, 22-42d and 0 42d in broilers exposed to heat stress from day 22 to day 42 - A) absolute FCR; B) relative difference of betaine and combinations of compound "A" and "B" to the negative control; C) relative difference of combinations of compound "A" and "B" to betaine; *a,b,c: different small letters indicate significant differences between means.

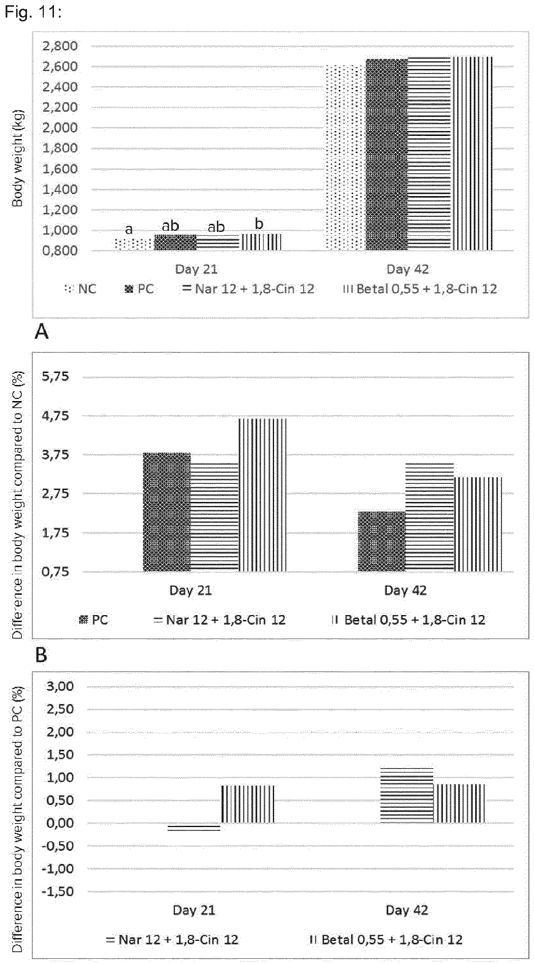

Fig. 11: Trial VI: Effect of betaine (reference substance) and combinations of compound "B" with compound "A" or "C" on body weight at days 21 and 42 in broilers exposed to heat stress from day 22 to day 42 - A) absolute body weight; B) relative difference of betaine and combinations of compound "B" with compounds "A" or "C" to the negative control; C) relative difference combinations of compound "B" with compounds "A" or "C" to betaine; *a,b,c: different small letters indicate significant differences between means.

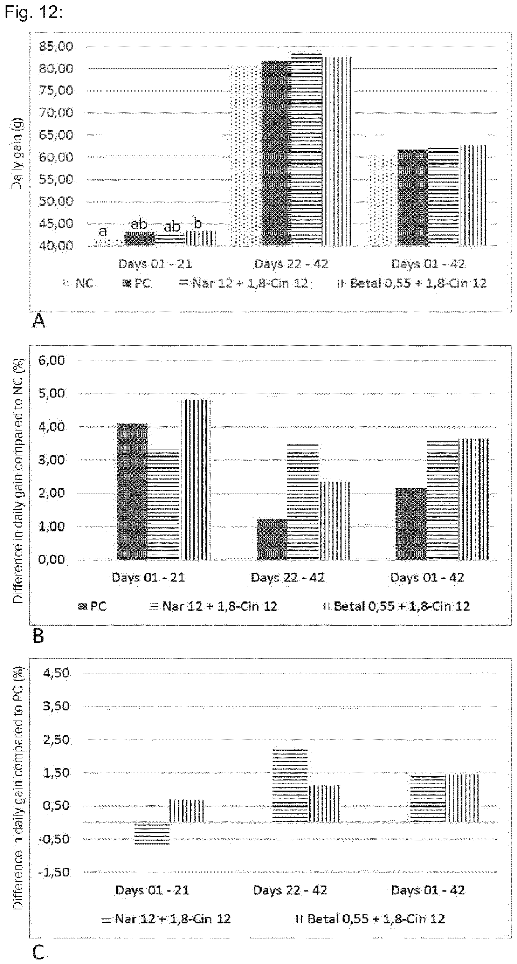

Fig. 12: Trial VI: Effect of betaine (reference substance) and combinations of compound "B" with compound "A" or "C" on daily weight gain in the periods 0-21 d, 22-42d and 0-42d in broilers exposed to heat stress from day 22 to day 42 - A) absolute daily weight gain; B) relative difference of betaine and combinations of compound "B" with compounds "A" or "C" to the negative control; C) relative difference combinations of compound "B" with compounds "A" or "C" to betaine; *a,b,c: different small letters indicate significant differences between means.

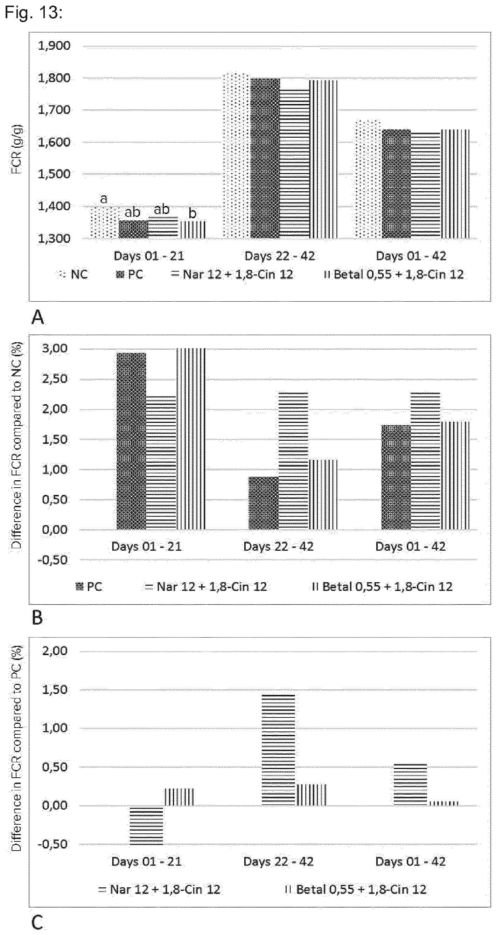

Fig. 13: Trial VI: Effect of betaine (reference substance) and combinations of compound "B" with compound "A" or "C" on FCR in the periods 0-21d, 22-42d and 0-42d in broilers exposed to heat stress from day 22 to day 42 - A) absolute FCR; B) relative difference of betaine and combinations of compound "B" with compounds "A" or "C" to the negative control; C) relative difference combinations of compound "B" with compounds "A" or "C" to betaine; *a,b,c: different small letters indicate significant differences between means

### DETAILED DESCRIPTION OF THE INVENTION

Specific terms as used throughout the specification have the following meaning.

The term "antibiotic-free" as used herein with respect to feeding an animal, a diet or a feed product, shall refer to the feeding of an animal with a feed product devoid of antibiotics. Though the animal may have been treated with antibiotics upon veterinary prescription, the regular diet would not contain additional antibiotics as growth enhancer. Thus, accumulation of such harmful substances as antibiotics and the like in persons who have consumed the meat or eggs of poultry is prevented. The compositions as described herein effectively improve the food conversion thereby substituting antibiotics in feed products. Thus, it is possible to increase the productivity of meat of good quality.

The term "biophysical characteristics" of an animal like livestock or poultry is herein understood as the biotic and abiotic function of an animal, or population, and includes particularly the factors that have an influence in their survival, development and evolution, in particular including factors improving the feed conversion efficiency in animals, e.g. as determined by *in vitro* or *in vivo* models. Such factors include e.g. intestinal membrane permeability, nutrient digestibility, (ileal) protein digestibility, nutrient transport, antimicrobial or antioxidative effects.

The "feed conversion efficiency" (FCE) as herein understood specifically refers to a measure of an animal's efficiency in converting feed mass into increased body mass (e.g. muscle or egg mass). The efficiency may be determined as the feed conversion rate (FCR), which is the mass of the food eaten divided by the body mass gain, all over a specified period. For example, an animal being fed with a feed additive designed for improved feed conversion efficiency may consume less food than an animal that received feed without such feed additive, producing a similar amount of meat. Typically, the feed conversion rate of poultry is in the range of 1.2 to 2.5, and the feed conversion rate of swine is in the range of 1.5 to 3.0, depending on the genetic breed. An improval (*i.e*., a reduction) in the feed conversion rate or a factor influencing the feed conversion efficiency may be determined, if the feed conversion rate is increased, or the factor is decreased, e.g., by at least 2%, preferably at least 2.5%.

There are direct factors influencing the feed conversion efficiency, e.g. including nutrient digestibility, (ileal) nutrient or protein digestibility, intestine membrane permeability, nutrient transport systems of the brush border membrane, or indirect factors, such as those improving the health status of the animal and reducing the energy and protein demand for immune reactions, including antimicrobial effects.

The term "intestine membrane permeability" is herein understood as a factor determining intestinal absorption of nutrients, such as by passing though a cellular membrane of the intestine. Such biophysical characteristics may be determined by the *ex vivo* model employing epithelial colorectal cells to test a change in permeability upon contact with specific substances. The term "nutrient transport systems" is herein understood as an active transport of nutrients, including e. g. glucose, peptides and amino acids, from the lumen of the small intestine across the brush border membrane into the enterocytes. Such nutrient transport systems are specific enzymes, including e.g. the sodium-dependent glucose transporter (SGLT1) and small peptide and amino acid transporter (PEPT1). Such biophysical characteristics may be determined by the *ex vivo* model employing epithelial colorectal cells to test a change in gene expression of the SGLT1 transporter enzyme upon contact with specific substances. The term "antimicrobial effects" as understood herein refers to the possible bacteriostatic effects of bacteria that are possibly pathogenic to monogastric animals, including poultry. Such biophysical characteristics may be determined by the *ex vivo* model employing bacterial cells to test a change in bacterial cell growth upon contact with specific substances.

The term "component" with regard to a feed additive is herein understood as a part of a composition, which may include one or more further compounds, components and excipients. The feed additive described herein is a composition specifically comprising at least an (essential) oil component comprising 1,8-cineole as a compound or an active compound, and at least one further component including the compounds (or active compounds) naringin and/or betalain, but may further include biological components, primarily phytogenic components and further excipients, including *e.g*., anorganic excipients.

Specifically, the 1,8-cineole component is an essential oil comprising 1,8-cineole in a specified amount. Exemplary plant materials used as a source of the 1,8-cineole compound as used herein, are any one or more of: thyme, rosemary, or eucalyptus

The oil component as described herein is specifically obtained by extracting an oil from a plant material e.g., by cold extraction or hot extraction techniques, employing aqueous and optionally an oily phase, so to obtain a w/o emulsion of the oil.

According to a specific example, the 1,8-cineole component is produced as follows: In a mixer employing spray technology, the essential oil component is sprayed on silica in any one of the ratios of 30:70 or 40:60 or 50:50 or 60:40 (w/w) and thoroughly mixed e.g., for at least 30 minutes.

Essential oils are typically extracted from plant materials through removal methods that are suited to the specific plant part containing the oils. Popular extraction methods include: steam distillation, solvent extraction, CO₂ extraction, maceration, enfleurage, cold press extraction, and water distillation.

According to a specific aspect, the production method employs the preparation of an o/w emulsion including the oil, and optionally a polymer, solubilizer and/or detergent, followed by spray-drying at controlled temperature to obtain a flowable dry oil component.

Essential oils can be provided in the microencapsulated form. Typically, the microencapsulation of an essential oil provides for its isolation from its surroundings *e.g*., isolating an oils from deteriorating effects of further substances in the aqueous phase *e.g*., in the gastrointestinal environment, retarding evaporation of the volatile oil, protection against friction and evaporation due to humidity and high temperature during the feed processing (pelleting) or improving the handling properties of the sticky material. In addition, the rate may be effectively controlled at which the oil leaves the microcapsule, as in the sustained or controlled release of the oil in the gastrointestinal tract, aiming at providing an effective amount of the active compounds in the intestines. Thereby the release of the oil and other components may be achieved in a synchronized way to achieve synergistic effects *in vivo.*

A wide range of materials and methods may be used for encapsulation to create the degree of durability and method of release suitable to the intended use. Nonlimiting polymeric exemplary materials suitable for use with the microencapsulation of oil may include natural polymers of eukaryotic or prokaryotic origin, e.g. including starch hydrolysates, like dextrins, modified starch, Gummi Arabicum, alginates, cellulose derivatives, like hydroxypropylcellulose, Na-carboxycellulose, methylcellulose, ethylcellulose, animal or plant proteins or protein hydrolysates, like gelatin, collagen, egg yolk, wheat protein, casein, milk protein, soy protein, pea protein, or mixtures thereof. Various physical and chemical methods of microencapsulation may be used, depending on the oil and the desired polymeric shell coating to be used. Conveniently, the essential oil is encapsulated by dehydrating an o/w emulsion by any suitable means, including spray drying, freeze drying, fluid bed drying, tray drying, adsorption, and combinations thereof. Preferably, the microencapsulated oil is produced by spray-drying an emulsion having an aqueous phase as defined above containing a polymeric encapsulation agent. The spray-drying parameters are dictated by the physical characteristics desired in the final microencapsulated oil. Such physical parameters include particle size, flow and water content.

The oil component typically has good flowability and can be distributed homogeneously throughout the composition. Conveniently, the oil component is a powder. Any suitable additive may be added to the oil *e.g*., a flow agent such as silicon dioxide, to increase the flowability of the oil.

The naringin component and/or the betalain component as described herein is specifically a plant material comprising the compounds naringin and betalain, respectively, in the specified amount. Specifically, the plant material is provided as particulate dry powder.

The term "powder" as used herein is specifically understood as a flowable material comprising a plurality of particles. The particles may have a smooth outer surface and/or a flattened morphology. In certain embodiments, the particulate plant material is a beige to dark brown powder with a characteristic smell.

The particulate plant material used herein is specifically obtained by grinding dry plant material to obtain a specific particle size, e.g. corresponding to flowable material, *e.g*., SiO₂ (powder), a flour and/or semolina.

The particles may have an average largest dimension of 250-500 µm. The typical particulate material has a particle size of min 95 % below 500 µm. Preferably, the particulate plant material has a mean particle size of 100- 350 µm.

The plant powder material can be derived from various portions of the plant, specifically fibrous plant materials may be used, *e.g.* including bark, roots, stalks, stems, leaves, fruits, peels, flowers, seeds, or combinations thereof.

Exemplary plant materials used as a source of the naringin component as used herein, are plant materials of plants of the family Rutaceae, such as citrus fruits, e.g., citrus fruit peels or pomace. Specifically, naringin can be extracted from such plant products and used as feed additive component, or the plant products can be used as such.

Exemplary plant materials used as a source of the betalain component as used herein, are plant materials from plants of the Caryophyllales, such as fruits, leaves, stems, and roots of plants e.g., beets. Specifically, betalains can be extracted from such plant products and used as feed additive component, or the plant products can be used as such.

According to a specific example, the naringin component and/or betalain component is admixed in a feed mixer to the essential oil component on silica and optionally with other excipients.

The moisture content of the feed additive or feed product described herein is typically less than 12 %, preferably less than 8 %.

The term "excipients" as used herein shall refer to additive components commonly used in feed compositions, e.g. phytogenic and/or inorganic feed additive components. Specifically, the feed additive and its additive components are understood as products used in animal nutrition for purposes of improving the quality of feed, or to improve the animals' performance and health. Feed additives are typically carefully selected which have no harmful effects, on human and animal health and on the environment.

In this regard, the term "feed" typically refers to any mixture of animal feed ingredients providing energy and nutrient requirements, e.g., protein, fat, carbohydrates, minerals and micronutrients. For example, the daily intake of poultry feed is typically between 50 - 250 g/head and day for a broiler for fattening. According to another example, the daily intake of swine feed, according to the physiological status, is typically between 50 - 1500 g/head and day. According to another example, the daily intake of ruminant feed, according to the species and the physiological status, is typically between 150 - 35000 g/head and day.

The feed additive described herein may specifically include excipients, such as further essential oils, dried herbs, spices and further excipients, including colors, flavoring substances, preservatives, or any substance needed to formulate the composition to the desired form, such as bulking, anti-caking agents, diluents, fillers, binders, disintegrants, adsorbents, or granulating agents. Typical excipients are for example rosemary leafs, juniper berries, psyllium hulls, wheat bran, limestone, SiO₂ or bentonites.

The term "flowable" as used herein shall specifically refer to a mixture of components in the powder form, including e.g. particulate material, which may flow. For example, a flowable mixture may flow through a funnel or hopper into another container under the influence of gravity. In the present invention, a flowable powder mixture is suitable for use with a device for mixture with feed material and pelleting. The term "flowable" is well known in the food and feed industry and has a clear meaning to the person skilled in the art.

A flowable mixture has several advantages in use, particularly on an industrial scale. The mixture may be handled, stored and transported relatively easily and energy-efficiently, as compared with, for example, solid materials that are not flowable. This advantage is particularly important in combination with the ability to avoid a liquefying step in the pelleting process.

The flowable mixture as used herein specifically comprises at least an (essential) oil component comprising 1,8-cineole, and at least one further component including naringin or betalain, and optionally other components and excipients, wherein the components are all mixed together without the inclusion of any substantial amount of liquid to form a dry mixture, which is optionally ground into a flowable, preferably pelletable powder.

Specifically, a 1,8-cineole component, fed in a mixture with at least one further component including naringin or betalain, provide for a phytogenic system that inherently improves the sustained release properties of the active compounds *in vivo*, to improve the biophysical characteristics as required. Preferably, any one or more or all of the 1,8-cineole component, the naringin component or the betalain component do not dissolve or dissolve only poorly in the stomach. The components of the feed additive composition can be sufficiently provided to the intestine and/or colon, so that the active compounds may concomitantly act on the biophysical characteristics.

Thus, it is possible to obtain synergistic effects in the intestines when using the feed additive or feed product described herein.

The term "poultry" as used herein shall specifically refer to domesticated fowl kept primarily for meat (broilers) and eggs (layers); including birds of the order Galliformes, e.g., the chicken, turkey, guinea fowl, pheasant, quail, and peacock; and Anserigormes (swimming birds,) e.g., the duck and goose.

The poultry may be fed with the same feed composition throughout the growth period, or at least during a period of at least 3 weeks, preferably at least 4 weeks to improve the efficiency of feed use or feeding efficiency, e.g. output per unit of feed. Significant differences may be found between control and experimental treatments in final body weight and weight gain at the entire growth periods till 28 days after hatching. While the feed intake may be almost equal between treatments, the feed efficiency, i.e. g feed/g weight gain may be significantly better for poultry and specifically chicken fed with the feed additive composition of the invention.

The term "swine" as used herein shall refer to stout-bodied short-legged omnivorous artiodactyl mammals (family Suidae) with a thick bristly skin and a long flexible snout; especially the domestic pig and the wild boar.

The feed described herein as used for poultry or pigs is specifically pelleted or mash.

Exemplary compositions of a feed additive composition described herein for use in breeding poultry or pigs are described in Example 1.

The term "ruminants" as used herein shall refer to ruminating mammals including e.g., cattle, goats, sheep, giraffes, yaks, deer, antelope, and some macropods (kangaroos).

The feed described herein as used for ruminants, such as cattle, specifically is pelleted, or a feed additive admixed to one or more of the following: grass-silage, corn-silage, soy bean meal, or bruised grain.

Exemplary compositions of a feed additive composition described herein for use in breeding ruminants, such as cattle, are described in Example 1.

Further examples provided herein are directed to the analytical methods to determine the active substances 1,8-cineole, naringin and betalain in a feed additive composition.

Further examples provided herein are directed to in vivo and ex vivo testing of the effect of the feed additive, such as
i. In vivo testing the effect on life span extension in C. elegans worms which are exposed to heat stress,
ii. Ex vivo testing the effect heat shock protein induction in CaCo2 cells;
iii. Ex vivo intestinal barrier function by transepithelial electrical resistance (TEER) in transwell CaCo2 cells;
iv. In vivo validation of beneficial effects of naringin on growth performance in broilers under heat stress and evaluation of a beneficial dose level;
v. In vivo validation of beneficial effects of combinations of naringin with different 1,8-cineole concentrations on growth performance, heat shock protein response and intestinal barrier integrity in broilers under heat stress; and
vi. In vivo validation of beneficial effects of the combinations of naringin with 1,8-cineole, or 1,8-cineole with betalain.

The foregoing description will be more fully understood with reference to the following examples. Such examples are, however, merely representative of methods of practicing one or more embodiments of the present invention and should not be read as limiting the scope of invention.

### EXAMPLES

### Example 1: Exemplary feed additives named "New Feed Additive Supporting Animal Welfare Under Heat Stress Conditions"

Exemplary compositions contain the following ingredients, admixed to a dry flowable mixture. Whereas the oil component is a specific lamiaceae oil or a mixture of lamiaceae oils (e.g., of Oregano, and/or Rosemary, and/or Thyme), standardized to the concentration of 1,8 cineole, the flavonoid component derives from citrusflavonoids, with standardized Naringin concentration, and the betalain component derives from Beetroot powder, with standardized betalain concentration.

**Table 1: Formula I; Composition of a new feed additive formulation for Poultry (Broilers, Layers, Turkey) and Pigs**

| Ingredients | Content (%, w/w) |
|---|---|
| Lamiaceae oil mixture, in total | ≥ 4,00 (4,00 - 16,0) |
| 1,8-cineole | ≥ 1,50 (1,50 - 6,00) |
| Citrus flavonoids, in total | ≥ 5.50 (5,50 - 11,0) |
| Naringin | ≥ 3,00 (3,00 - 6,00) |
| Excipients | To 100% |

The exemplary feed additive formula I contains 1.50 % 1,8-cineole (w/w); and an effective ratio of 0.65:1 (w/w 1,8-cineol per naringin).

**Table 2: Formula II; Composition of a new feed additive formulation for Poultry (Broilers, Layers, Turkey) and Pigs**

| Ingredients | Content (%, w/w) |
|---|---|
| Lamiaceae oil mixture, in total | ≥ 4,00 (4,00 - 16,0) |
| 1,8-cineole | ≥ 1,50 (1,50 - 6,00) |
| Beetroot powder, in total | ≥ 10.0 |
| Betalain | ≥ 0,125 (0,125 - 0,25) |
| Excipients | To 100% |

The exemplary feed additive formula II contains 1.50 % 1,8-cineole (w/w); and an effective ratio of 22:1(w/w 1,8-cineol component per betalain).

**Table 3: Formula III; Composition of a new feed additive formulation for Ruminants**

| Ingredients | Content (%, w/w) |
|---|---|
| Lamiaceae oil mixture, in total | ≥ 1,00 (1,00 - 4,00) |
| 1,8-cineole | ≥ 0,375 (0,375 - 1,50) |
| Citrus flavonoids, in total | ≥ 1,30 (1,30 - 2,60) |
| Naringin | ≥ 0,75 (0,75 - 3,00) |
| Glycerol (1,2,3-Propanetriol) | ≥ 50,0 |
| Excipients | To 100% |

The exemplary feed additive formula III contains 0,375 % 1,8-cineole (w/w); and an effective ratio of 0.5:1 (w/w 1,8-cineol per naringin).

**Table 4: Formula IV; Composition of a new feed additive formulation for Ruminants**

| Ingredients | Content (%, w/w) |
|---|---|
| Lamiaceae oil mixture, in total | ≥ 1,00 (1,00 - 4,00) |
| 1,8-cineole | ≥ 0,375 (0,375 - 1,50) |
| Beetroot powder, in total | ≥ 5.00 |
| Betalain | ≥ 0,033 (0,033 - 0,066) |
| Glycerol (1,2,3-Propanetriol) | ≥ 50,0 |
| Excipients | To 100% |

The exemplary feed additive formula IV contains 0,375 % 1,8-cineole (w/w); and an effective ratio of 12:1 (w/w 1,8-cineol per betalain)

The feed additive called "New Feed Additive Supporting Animal Welfare Under Heat Stress Conditions" contains
a) naringin at an effective ratio within the range of 0.2:1 - 1.5:1, in particular 0.65:1 (w/w, 1,8-cineole per naringin); and/or
b) betalain at an effective ratio within the range of 4:1 - 64:1, in particular 22:1 (w/w, 1,8-cineole per betalain).

The formula is tested in the test systems described herein to show a synergistic effect of combinations of the individual compounds.

For example, the synergistic effect is defined as higher growth performing efficacy compared to a standard reference additive, when used at a recommended dose level.

According to the example, the dosage of the "New Feed Additive Supporting Animal Welfare Under Heat Stress Conditions" is described in a range of 200 to 400 mg/kg or in a range of 200 to 400 mg/kg of complete feed.

Moreover, in the in vivo test systems (animal trials) a usually recommended dose of betaine (500 mg/kg of complete feed) is carried along as a positive control group. This approach serves to prove the higher efficacy of the "New Feed Additive Supporting Animal Welfare Under Heat Stress Conditions" compared to a frequently applied commercial solution.

### Example 2: Description of analytical methods and trial methodologies:

### Description of the analytical active substance evidence

1. 1,8 Cineole (compound "A")
2. Naringin (compound "B")
3. Betalain (compound "C")

### Description of trial methodology and results

4. Trial I: Testing of life span extension in *C. elegans* worms
5. Trial II: In vitro testing of heat shock protein induction in CaCo2 cells
6. Trial III: In vitro testing of intestinal barrier function by transepithelial electrical resistance (TEER) in transwell CaCo2 cells
7. Trial IV: In vivo validation of beneficial effects of the naringin (Compound "A") on growth performance in broilers under heat stress and evaluation of the optimum dose level
8. Trial V: In vivo validation of beneficial effects of combinations of naringin (Compound "B") with different 1,8-cineole concentrations (Compound "A") on growth performance, heat shock protein response and intestinal barrier integrity in broilers under heat stress
9. Trial VI: In vivo validation of beneficial effects of the combinations of naringin with 1,8-cineole (AB), or 1,8-cineole with betalain (AC).

### Description:

### 1,8-CINEOL analysis

### GC MS

Gas chromatography analysis was carried out using a Focus GC coupled to a DSQII MS purchased from Thermo (Waltham, MA, USA). The injection (injection volume 1 µl) was performed in the splitless mode at the injection temperature of 240 °C. Separation was carried out on an Rxi-5ms fused silica column (30 m × 0.25 mm i.d.; 0.25 µm film thickness) from Restek (Bad Homburg, Germany) at constant flow. The oven program started at 50 °C and the temperature was increased in the first step to 190 °C at 5 °C min-1 and in the second step to 300 °C (held for 5 min) at 30 °C min-1. Helium (4.6) was used as carrier gas with a column flow rate of 1.0 ml min-1.

The mass spectrometer was operated in the selected ion monitoring (SIM) mode with the selected ions m/z 154, m/z 139, m/z 111, m/z 108. The ion source temperature was adjusted to 240 °C and transfer line temperature configured to 300°C.

### Naringin analysis

### LC MS

The experiment was performed on an Agilent Series 1100 HPLC system. The separation column was a Poroshell 120 EC-C18 Eclipse (50 × 3 mm ID, 2.7 µm particle size) obtained from Agilent. The mobile phase consisted of a gradient of water and acetonitrile, both with 0.1% formic acid. Initial conditions were 15% acetonitrile for 5 minutes, then the gradient was run to 20 % acetonitrile within 5 minutes. 100 % acetonitrile within the next 5 minutes, and finally to 15 % acetonitrile kept for another 5 min. The total chromatographic run time was 20 minutes. The flow rate was set to 0.8 mL min⁻¹.

MS detection was carried out on an Agilent 6520 QTOF in the negative ion mode. The following ion source conditions were used: drying gas temperature 350 °C, drying gas flow 10.5 L min-1, nebulizer pressure 45 psi, fragmentor voltage 125 V and capillary voltage 3750 V.

### BETALAIN analysis

### LC MS (Pires Goncalves et al. 2012)

Reversed-phase chromatography was performed in a Waters (Milford, MA) 600 system equipped with a UV-Vis detector (dual-wavelength, Waters 2489) and a Jupiter-15 (300 Å, 15 µm, 250 × 21.2 mm, Phenomenex, Torrance, CA) C₁₈ column. Gradients were formed between two helium-degassed solvents: solvent A: water with 1% v/v HOAc; linear gradient from 5% to 20% B: in 60 min at 25 °C, flow rate: 10 mL/min.

A Bruker Daltonics Esquire 3000 Plus was used for the ESI-MS analyses. The vaporizer temperature was 325 °C and the voltage was maintained at 4.0 kV. The sheath gas was nitrogen, operated at a pressure of 26 psi (6.0 L/min). Compounds were ionized in the positive mode.

### Description of trial methodologies

### Trial I: Testing of life span extension in C. elegans worms

For this investigation a specific assay with *C. elegans* has been developed. Under regular temperature conditions *C. elegans* has a life span of about 14 days, including all larval stages. Worms reach their adult state after 3 to 4 days. Increasing temperature to 37°C reduces the remaining life span of *C. elegans* drastically to 10 to 15 h. Consequently, in this assay the elongation of life span by control substances (Ascorbic acid and betaine) and numerous other phytogenic compounds (Mint oil, Eucalyptus oil), as well as some of their purified active constituents (Piperin, Capsaicin, Gingerol, Eugenol) has been screened. All tested substances have been used in concentrations (mg/L) which could be transferred later to dietary concentrations (mg/kg) of these substances in complete diets.

**Table 5: Trial I: Concentration of compounds A, B and C and other active ingredients**

| Ingredients | | Concentration (mg/L) | Equivalent concentration in feed (mg/kg) or water (mg/L) |
|---|---|---|---|
| Ascorbic acid | | 100 | 100 (water) |
| Betaine | | 500 | 500 (feed) |
| Citrus flavonoids | | 250 | 250 (feed) |
| | • thereof (B) | 50 | 50 (feed) |
| Lamiaceae oil mixture | | 25 | 25 (feed) |
| | • thereof 1,8-cineole (A) | 7.5 | 7.5 (feed) |
| Beetroot powder | | 250 | 250 (feed) |
| | • thereof Betalain (C) | 0.6875 | 0.6875 (feed) |
| Mint oil | | 50 | 50 (feed) |
| Eucalyptus oil | | 50 | 50 (feed) |
| Eugenol | | 50 | 50 (feed) |
| Pepper oleoresin | | 50 | 50 (feed) |
| Capsicum oleoresin | | 50 | 50 (feed) |
| Ginger oleoresin | | 50 | 50 (feed) |

### C. elegans maintenance:

*C. elegans* wildtype strain N2, variation Bristol were obtained from the *C. elegans* Genetics Center, CGC (University of Minnesota, MN, USA). Nematodes were maintained on nematode growth medium (NGM) agar plates seeded with *E. coli* OP50 at 20° C according to standard protocols (Brenner S, 1974, The genetics of Caenorhabditis elegans*.* Methods such as freezing nematodes and obtaining synchronous populations using a bleaching method with hypochlorite treatment of egg-laying adults were also performed according to standard protocols (Stiernagle T, 2006; Maintenance of *C. elegans.* WormBook 1-11).

### Treatment of nematodes with reference substances (ascorbic acid, betaine) and the main ingredients of the "New Feed Additive Supporting Animal Welfare Under Heat Stress Conditions"

Synchronous nematodes were raised in liquid culture using NGM liquid and packed *E. coli* HT115 according to Stiernagle. Carbenicillin was added to the NGM liquid in order to inactivate *E. coli.* A volume of 56 µl of NGM liquid was dispensed in each well of a 96-well microplate, to which 10 µl M9-buffer containing 10 synchronized L1 larvae were added. L1 larvae were maintained shaking at 20 °C and reached the adult stage within 3 days. All control substance (ascorbic acid, betaine) and test substances (Compounds "A" and "B") were prepared as stock solutions in M9-buffer and sonicated for 5 min. Stock solutions (10-fold) had the following concentrations (Ascorbic acid 500/1000 mg/L, Betaine 5000 mg/L, Compound "A" 75mg/L, Compound "B" 500 mg/L, Compound "C" 6,88 mg/L). 7 µl of each extract stock solution was added to the incubation medium to reach a final concentration of 100 mg/L (Ascorbic acid), 500 mg/L (Betaine), 7,5 mg/L (Compound "A"), 50 mg/L (Compound "B") and 0,6875 mg/L (Compound "C"). Control nematodes were always treated with identical volumes of M9-buffer instead.

### Determination of survival under heat stress

After incubation of young adult N2 nematodes for 48 h at 20 °C in the presence or absence of the control additives (ascorbic acid, betaine) and the New Feed Additive Supporting Animal Welfare Under Heat Stress Conditions components "A" and "B"

Survival was determined using a microplate thermotolerance assay as described (Gill MS, Olsen A, Sampayo JN, Lightgow GJ, 2003; Free Radic Biol Med 35:558-565). In brief, nematodes were washed off the wells with M9-buffer/Tween^{®}20 (1% v/v) into 15 ml tubes followed by additional three washing steps. In each well of a black 384-well low-volume microtitre plate 6.5 µl M9-buffer/Tween^{®}20 (1% v/v) solution was added. Subsequently, one nematode was dispensed in 1 µl M9 buffer under a stereo-microscope (Breukhoven Microscope Systems) into each well and mixed with 7.5 µl SYTOX green to reach a final concentration of 1 µM. To prevent water evaporation the plates were sealed with Rotilab sealing film and covered with a lid. Heat shock (37°C) was induced and fluorescence was measured with a Fluoroskan Ascent microtiter plate reader (Thermo Labsystems, Bonn, Germany) every 30 min. To detect SYTOX green fluorescence, excitation wavelength was set to 485 nm and emission was measured at 538 nm.

To determine the survival time for each nematode an individual fluorescence curve was generated. Time of death was defined as one hour after an increase in fluorescence over the baseline level was observed and was verified by touch provocation first. From the individual times of death Kaplan-Meier survival curves were drawn.

### Results: testing of life span extension in C. elegans worms (Trial I)

To investigate heat stress protective effects of both reference substances (Ascorbic acid, betaine) and of phytogenic compounds, including compound "A", "B" and "C" of the "New Feed Additive Supporting Animal Welfare Under Heat Stress Conditions", life span extension in *C. elegans* was tested under heat stress conditions (Figure 1). Tested concentrations of the reference substances and of the phytogenic substances were chosen to reflect final dietary concentrations in animal diets (e.g. the reference substance betaine is used in animal diets in concentrations from 250 to 1000 mg/kg of diet; in the *C. elegans* model betaine was tested at a level of 500 mg/L = 500 mg/kg = 500 mg/kg). Both reference substances (ascorbic acid and betaine) increased life span for 0.5 to 1.5 hours. Compounds "A" (Figure 1D), "B" (Figure 1C) and "C" (Figure 1E) at the tested concentrations increased life span by 0,5 to 1,5 h.

Interestingly, comparative examples using pungent substances and cooling essential oils, frequently recommended against negative consequences of heat stress, and partially used in commonly available feed additive products, showed not benefits on life span extension (Figure 2 F, G, H, I, J, K).

### Trial II: In vitro testing of heat shock protein induction in CaCo2 cells

For this investigation a specific assay with CaCo2 monolayer cells has been developed. CaCo2 cells are regularly incubated at 37°C. Increasing the temperature to 41°C causes a heat shock response in these cells (induction of heat shock proteins) to counteract cellular damage and to initiate repair mechanisms. Consequently, in this assay the potential of betaine (reference substance) and of compounds "A" and "B" has been studied in a dose-dependent manner to support and increase natural heat shock response.

### Study with CaCo2 cells

### Materials

MEM with Earle's salts, fetal bovine serum (FBS), penicillin/streptomycin and trypsin-EDTA were purchased from Biochrom GmbH (Berlin, Germany). Entero-STIM Intestinal Epithelium Differentiation Medium and MITO+ Serum Extender were obtained from Corning (Wiesbaden, Germany) and cell culture plates were purchased form Greiner Bio-One International GmbH (Kremsmünster, Austria). RNeasy Mini Kit was obtained from Quiagen (Hilden, Germany), i Script cDNA Synthese Kit and iQ SYBR Green Supermixture from Bio-Rad (Munich, Germany) and oligo dT-primers from Eurofins Genomics (Ebersberg, Germany).

### Cell culture and differentiation of CaCo2 cells

Human CaCo2 cells (DSMZ, Braunschweig, Germany) were maintained in MEM with Earle's salts supplemented with 10% FBS and 100 U/mL penicillin/100 µg/mL streptomycin and grown at 37 °C in a humidified atmosphere (≥ 95%) with 5% CO₂. The cells were seeded in 12-well plates at 1.2 × 10⁶ cells per well to reach confluency the next day. The cells were further maintained in Entero-STIM Intestinal Epithelium Differentiation Medium supplemented with 100 U/mL penicillin/100 µg/mL streptomycin and 0.1% MITO+ Serum Extender and the medium was changed daily. The experiment was carried out on day 5 when the cells were completely differentiated.

### Induction of heat stress

To analyze the influence of betaine (reference substance) and compounds "A" "B" and "C" on the expression of the heat shock protein HSP70, the substances were added to the cells on day 4 and the cells were incubated with the extract overnight for 15 hours. The test substances were dissolved in complete differentiation medium and diluted to the following final concentrations: Betaine (250/500/1000/1500 mg/L); Compound "A", (7,5, 15, 30 and 60 mg 1,8-cineole/L); Compound "B", (6, 12, 18, 24, 30 and 60 mg Naringin/L); Compound "C", (0,55, 1,10, 2,20 mg Betalain/L), respectively. To induce heat stress, the samples were incubated at 41 °C for 1 hour, while control samples were incubated at 37 °C for the same time.

### Detection of HSP70 mRNA expression by real-time PCR

The mRNA expression of HSP70 was measured quantitatively by real-time PCR (C1000 Thermal Cycler and CFX96 Real-Time System, Bio-Rad, Munich, Germany). Total RNA was isolated with RNeasy mini kit, followed by the transcription of 50 ng of total RNA into cDNA with the i Script cDNA Synthesis Kit (end volume: 20 µL) and the real-time PCR with the iQ SYBR Green Supermix according to manufacturer's instructions. Briefly, for real-time PCR 2 µL of cDNA was added to 18 µL master mixture (10 µL iQ SYBR Green supermix (2x), 2 µL primer [3 pmol/µL], 6 µL nuclease-free water). DNA denaturation and polymerase activation for 3 min at 95 °C was followed by 40 PCR cycles. One amplification cycle is divided into three parts: denaturation at 95 °C for 15 sec., annealing and extension at 60 °C for 60 sec. followed by a plate read after each cycle. Finally, a melt curve analysis was done by gradually increasing the temperature to 95 °C, to exclude the formation of primer-dimers. The detected c_{T} values were used for the calculation of the relative mRNA expression levels via the 2-^{ΔΔc}_{T} method (ZITAT LIVAK). Differences in cDNA amounts were normalized to the expression of β-actin mRNA. Statistical analysis was carried out in Graphpad Prism (version 6.02) using unpaired t test. The following primers were used for the amplification: β-actin forward: 5'-GCG GGA AAT CGT GCG TGA CAT T-3' (SEQ ID NO:1); β-actin reverse: 5'-GAT GGA GTT GAA GGT AGT TTC GTG-3`(SEQ ID NO:2); HSP70 forward: 5'-CTA GCC TGA GGA GCT GCT GCG ACA G-3' (SEQ ID NO:3); HSP70 reverse: 5'-GTT CCC TGC TCT CTG TCG GCT CGG CT-3' (SEQ ID NO:4).

### Results of in vitro testing of heat shock protein induction in CaCo2 cells (Trial II)

To investigate the benefits of compounds "A", "B" and "C" on the additional induction of heat shock response differentiated CaCo2 cells were exposed to increasing levels of compounds "A", "B" and "C", representing the above mentioned dose range applied in animal diets (Figure 3 B, C, D). As a reference substance, the frequently used anti heat stress feed additive betaine was tested accordingly (Figure 3A). Betaine showed neither a clear dose-response on additional heat shock protein 70 induction over the control. Whereas 100, 250 and 1000 mg/L betaine even decreased heat shock protein 70 induction compared to untreated control cells (-7 to -19%), 500 and 2000 mg/L resulted in a non-significant increase in heat shock protein 70 response (+10 to +12%) (Figure 3 A). In contrast, all three compounds of the "New Feed Additive Supporting Animal Welfare Under Heat Stress Conditions" resulted in clear benefits over the control and indicated either a dose response or exceeding of optimum concentrations (Figure 3 B, C, D). For compound "B" already the lowest Naringin concentration tested, resulted in a clear but not significant increase of HSP 70 induction compared to untreated control cells (+16%). A significant increase in HSP 70 induction nearly at the same level could be observed for Naringin concentrations between 12 to 30 mg/L of compound "B" (+24 to +27%). In contrast, 60 mg Naringin /L led to an adverse effect (-23%), indicating exceeding of the optimum dose range. Similarly already 7,5 mg 1,8 Cineole/L from compound "A" resulted in a 15% increase in HSP 70 induction compared to control cells. 15 and 30 mg 1,8-Cineole/L from compound "A" increased HSP 70 gene expression by 22 and 24%, respectively. The highest dose tested (45 mg 1,8-Cineole/L) increased HSP 70 expression even by 44%. However, due to price reasons of raw materials this concentration cannot be considered in a product formulation for a "New Feed Additive Supporting Animal Welfare Under Heat Stress Conditions". The highest concentration tested was in the range, reported from Türk et al. (2016). To test if there are additional effects of the single compounds, as an example HSP 70 induction by compound"A" and "C" alone, as well as of the combination of both substances is given in Figure 3 E. At the tested levels of substance "A" and "C" alone with 20.8% and 21,1 % a nearly significant or significant increase of HSP 70 expression could be achieved, which was at a comparable level, as reported in Figure 3 C and D. The combination of both substances nearly doubled HSP 70 induction to 40,2% and clearly showed the improved effect of both substances (Figure 3 E).

### Trial III: In vitro testing of intestinal barrier function by transepithelial electrical resistance (TEER) in transwell CaCo2 cells

In order to test, if the compounds "A", "B", and "C" help to support intestinal barrier integrity under heat stress conditions experiments with transwell CaCo2 cell layers have been performed. In a sterile, biosafety level 2 cell culture hood 0.8 mm pore PET transwell inserts were placed into a 24-well plate (4 wells per condition) All materials entering were sterilized with ethanol before.

Cell culture medium containing DMEM medium supplemented with 4.5 g/L glucose, 15% fetal bovine serum, and 1% Pen Strep, was prepared and heated to 37 °C in a water bath.

200-400 µl of the cell solution of human epithelial colorectal adenocarcinoma (Caco-2) cells in cell medium were placed the upper (apical) chamber of the transwell insert, at a density of 1.5 × 105 cells/cm2. The lower (basolateral) chamber was filled with 700 - 900 µl of cell medium.

Cells were cultured at 37 °C, 5% CO2, and 95% humidity for 16 days, and medium in the upper and lower chamber was replaced every 4 days. Following this incubation cells were incubated for additional 24h with tissue culture medium, containing compounds "A", "B" or "C" were added at levels comparable to Trial II. After this incubation time, basal TEER was measured according to Ghaffarian and Muro 2013. Subsequently, temperature was increased to heat stress conditions (42°C) and TEER measurement was continued for up to 6 h. From the comparison of TEER values of untreated control cells, carried along in every experiment were compared with those of cells treated with compounds "A", "B" or "C" conclusions on protective effects of the compounds on intestinal barrier function could be drawn.

### Results of In vitro testing of intestinal barrier function by transepithelial electrical resistance (TEER) in transwell CaCo2 cells (Trial III)

Since betalain could been shown for the first time to improve HSP 70 induction in CaCo2 cell monolayers (Figure 3 D), its protective effects on intestinal barrier integrity have been examined. It could be shown that betalain pretreatment of CaCo2 cells grown in transwell culture plates led to a significantly lower decrease of TEER under heat stress conditions compared to untreated control cells (Figure 4). From this fact, it could be concluded for the first time, that betalain unrolls protective effects on intestinal barrier integrity.

### TRIAL IV: In vivo validation of beneficial effects of the naringin (Compound "B") on growth performance in broilers under heat stress and evaluation of the optimum dose level

Beneficial effects of compound "B" have not yet been reported to improve animal welfare (improved performance, heat shock response and intestinal barrier integrity) in vivo. Since compound "B" showed a beneficial response in all in vitro tests, the first in vivo trial consequently aimed to investigate beneficial effects of this compound on performance and heat shock response in broilers under heat stress. Betaine, frequently used as an additive, improving performance of animals under heat stress was carried along in this trial as a positive control group.

### Trial design

1152 one-day old Ross 308 male broilers from a total of 1400 were used in the experiment. Any day-old broilers, showing signs of health problems, injury, or of being too small or in poor condition were excluded in the selection process. Broilers were sexed at the hatchery. All day-old chicks were individually weighted and grouped according to weight. The birds were then assigned to the 36 pens (6 repetitions of 6 treatments), and each pen initially consisted of 34 broilers. On day 4 of the experiment in each pen, the weakest 2 broilers were removed and all pens were equated to 32 birds per pen by negative selection. Birds of the negative control group were fed the basal diet without any further phytogenic additive. The reference group was fed a diet providing 500 mg/kg feed betaine from a natural betaine source (Actibeet^{®}, Agrana). To the diets of groups 3, 4, 5 and 6 the phytogenic premixes with compound "B" were added to achieve final dietary Naringin concentrations of 12/18/24/30 mg/kg of feed.

**Table 5: Trial IV: Experimental treatment groups**

| Treatment | Betaine (positive control), mg/kg feed | Naringin (compound B), mg/kg feed |
|---|---|---|
| T1 | - | - |
| T2 | 500 | - |
| T3 | - | 12 |
| T4 | - | 18 |
| T5 | - | 24 |
| T6 | - | 30 |

### Animal housing and management

The trial was carried out at the Delacon Research Facility (Stošíkovice na Louce, Czech Republic). Birds were kept in floor pens, each having an area of 2,1 m² (1,65 × 1,275 m; 2,03 m² of usable area = not counting the space occupied with the feeder), with fresh wood shavings as bedding material. Stocking density at the end of the trial was 14,78 chicks/m² (38,7 kg/m²). The building was supplied with artificial, programmable lights, automated central heating and forced ventilation. Temperature was set according to the breeders' recommendations from day 1 to day 21 of age. The onset of heat stress was on day 22 of age. Thereby a cyclic heat stress regime was applied. The heat stress period was from 09:00 in the morning until 17:00 in the afternoon. The average house temperature in this period was 34 °C. The "cooling night period" was from 19:00 in the evening until 07:00 in the morning. The average room temperature in this period was 26 °C. The time intervals from 07:00 to 09:00 in the morning and from 17:00 to 19:00 in the evening was used to gradually increase or decrease the temperature to 34°C and 26°C, respectively. In order to increase the humidity of the chicken house under the heat stress regime, towels on the heat panels were moistened 5 times per hour during the heat stress period. Relative humidity was maintained at about 70-80%.

### Experimental diets

Birds were fed a basal diet, based on wheat, corn, and soybean meal. For each feeding period (starter/grower, grower/finisher) feed was calculated to be iso-nutritive (Table 6). Dietary nutrient concentrations were calculated to fulfill the current recommendations of the broilers according to the breeders' recommendations. According to the applied 2-phase-feeding, a starter/grower diet was offered from days 1 to 21 and a grower/finisher diet from days 22 to 42. The composition of the diets, used in trials IV, V, VI, for the single phases and the calculated nutrient contents are given in Table. All diets were offered to the birds as pelleted feed.

**Table 6: Trials IV, V, VI: Composition and calculated analyses of the experimental diets**

| Feed formulas | | | |
|---|---|---|---|
| **Raw material** | **Unit** | **A) Starter/Grower** | **B) Grower/Finisher** |
| Wheat | % | 31,750 | 34,190 |
| Corn | % | 23,500 | 21,500 |
| Rape meal | % | 4,000 | 4,500 |
| Wheat flour | % | 2,500 | 4,500 |
| Maizegerm | % | | 1,500 |
| Soya meal | % | 29,000 | 25,000 |
| Animal fat | % | | 4,000 |
| Hydrolysed protein | % | 3,000 | |
| Soya oil | % | 1,000 | |
| L-Lysine HCl 98 | % | 0,370 | 0,300 |
| L-Threonine 98 | % | 0,070 | 0,100 |
| DL-Methionine liquid | % | 0,460 | 0,380 |
| Limestone | % | 1,500 | 1,300 |
| Salt | % | 0,200 | 0,230 |
| Monocalciumphosphate | % | 1,000 | 0,800 |
| Sodium carbonate | % | 0,150 | 0,200 |
| Vitamin and mineral premixture | % | **0,500** | **0,500** |
| **trial premixture with active compounds A,B,C** | % | **1.000** | **1.000** |
| Total | % | 100,00 | 100,00 |
| Calculated nutrients | | | |
| Dry matter | g/kg | 889,614 | 892,194 |
| MEp | MJ | 12,145 | 13,023 |
| Crude protein | g/kg | 226,606 | 200,375 |
| Fibre | g/kg | 27,203 | 27,776 |
| Fat | g/kg | 44,392 | 74,913 |
| Ash | g/kg | 60,557 | 53,039 |
| Lysine | g/kg | 14,433 | 12,077 |
| Methionine | g/kg | 6,967 | 5,915 |
| Met+Cys | g/kg | 10,475 | 9,508 |
| Threonine | g/kg | 8,929 | 8,127 |
| Arginine | g/kg | 14,405 | 12,462 |
| Ca | g/kg | 9,243 | 8,039 |
| P non phytate | g/kg | 3,341 | 2,907 |
| Na | g/kg | 1,920 | 1,578 |
| Cl | g/kg | 1,800 | 1,960 |

The premixes were prepared at the Delacon Biotechnik GmbH facility in Steyregg (Austria). They were prepared to provide the intended final dietary concentrations at an inclusion level of 0.1% (1.0 mg/kg). The concentration of active substances in the premixes was done immediately after preparation of the premixes.

The production of the complete pelleted diets with the addition of the premixes to the diets was carried by the feedmill Biosta s.r.o. Blučina (Czech Republic).

Samples were taken directly after manufacturing. 1 kg feed of each treatment and period will be stored at the trial facility at cool and dry conditions till the approval of the final report.

Diets were analyzed for nutrient content at Zemědělská oblastni laboratoř, Chotýšany. Diets were analyzed for dry matter, crude protein, crude fat, ash, sugar, starch, Ca, and P. Moreover, the active ingredients were analysed in the diets.

### Tissue sampling

On day 21 and 42 of the experiment 2 chickens of each repetition was killed for collecting liver and jejunum to measure gene expression.

### Statistical analysis of the data

The statistical analyses were performed with the software package SAS. After checking the homogeneity of the data, means were compared by the usual test procedures (Tukey test). Statistical significance was declared at P ≤ 0.05, with 0.05 <P ≤ 0.10 considered as a near-significant trend.

### Results of the in vivo validation of beneficial effects of the (Compound "B") on growth performance in broilers under heat stress and evaluation of the optimum dose level (Trial IV)

Table 7 shows the analysed concentrations of naringin in the diets of Trial IV. In the diets recovery of the active substance Naringin was nearly 100%, thus the specified contents appear also in the final diets. A similarly high recovery rate could be also analysed for the reference substance betaine.

**Table 7: Trial IV- Betaine and Naringin content of the trial diets**

| Treatment | Betaine (positive control) mg/kg feed | | Naringin (compound "B") mg/kg feed | |
|---|---|---|---|---|
| | analysed | expected | analysed | expected |
| T1 | - | - | - | - |
| T2 | 470 | 500 | - | - |
| T3 | - | - | 12.3 | 12 |
| T4 | - | - | 17.4 | 18 |
| T5 | - | - | 24.2 | 24 |
| T6 | - | - | 29.4 | 30 |

Since positive effects of compound "B" (Naringin) on animal welfare aspects under heat stress conditions (e.g. protection of intestinal barrier function, linked to an increase in performance) have not been reported yet, the aim of the trial was to evaluate dose-dependent effects of compound "B" versus a standard dose (500 mg/kg) of the reference substance betaine. Already in the pre-heat stress period (d 0 to 21), both the reference substance (+4.9%) and all doses (12, 18, 24 and 30 mg/kg diet) of compound "B" (+2.79 to + 13%) resulted in beneficial effects on body weight and daily body weight gain. Compared to the negative control this effect was even significant (+13%) for the lowest compound "B" dose tested (12 mg/kg) (Figures 5 and 6). The positive effect of the reference substance and of compound "B" on body weight development and daily gain continued also under heat stress conditions (d22 to 42) (Figures 5 and 6). However, under heat stress the effects of the reference substance betaine on body weight development were 2 to 5% higher compared to those of compound "B" (Figures 5 and 6). In contrast to the pre heat stress period a clear dose-response of compound "B" could be observed under heat stress (Figures 5 and 6). Due to the fact that additives against negative effects of heat stress are fed through the entire mast period, the strong preconditioning effect of already the lowest dose of compound "B" was maintained until the end of the trial. At day 42 birds of the groups treated with the reference substance (+170g) and with compound "B" (+80 to + 140g) had distinctly higher body weights compared with birds of the untreated control (Figures 5 and 6). The highest final body weight compared (+140 g) to the negative control could be obtained with the lowest compound "B" dose. Analogous results as found for body weight development could also be observed for feed conversion ratio (Figure 7).

### Trial V: In vivo validation of beneficial effects of combinations of naringin (Compound "B") with different 1,8-cineole concentrations (Compound "A") on growth performance, heat shock protein response and intestinal barrier integrity in broilers under heat stress

In the dose response trial for compound "B" (Trial IV) beneficial effects on the performance of the broilers under heat stress could be observed. It turned out that the lowest dose of 12 mg/kg Naringin complete diet resulted in performance values (body weight/weight gain/FCR) which were slightly better than in the betaine reference group. Higher concentrations of compound "B" did not result in further benefits for growth performance. In the dose response trial for compound "B" beneficial effects on the performance of the broilers under heat stress could be observed. Consequently in this trial the additional value of adding 1,8-Cineole (compound "A") in a dose-dependent manner (6/12/24 mg/kg feed) to compound "B" at constantly 12 mg Naringin/kg diet was evaluated. The addition of 500 mg/kg betaine alone or 500 mg betaine + 12 mg 1,8-Cineole/kg served as reference groups.

1224 one-day old Ross 308 male broilers from a total of 1400 were used in the experiment. Any day-old broilers, showing signs of health problems, injury, or of being too small or in poor condition were excluded in the selection process. Broilers were sexed at the hatchery. All day-old chicks were individually weighted and grouped according to weight. The birds were then assigned to the 36 pens (6 repetitions of 6 treatments), and each pen initially consisted of 34 broilers. On day 4 of the experiment in each pen, the weakest 2 broilers were removed and all pens were equated to 32 birds per pen by negative selection.

Birds of the negative control group were fed the basal diet without any further phytogenic additive. The reference groups 2 and 6 were fed a diet providing 500 mg/kg betaine from a natural betaine source (Actibeet^{®}, Agrana) or 500 mg/kg betaine from a natural betaine source (Actibeet^{®}, Agrana) + 12 mg/kg 1,8 Cineole (compound "A"). To the diets of groups 3, 4 and 5 the phytogenic premixes were added providing 12 mg/kg of Naringin plus 6, 12 or 24 mg 1,8-Cineole/kg (Table 8).

**Table 8: Trial V: Experimental treatment groups of the validation trial**

| Treatme nt | Betaine (positive control), mg/kg feed | Naringin (compound B), mg/kg feed | 1,8-cineole (compound A) mg/kg feed |
|---|---|---|---|
| T1 | - | - | - |
| T2 | 500 | - | - |
| T3 | - | 12 | 6 |
| T4 | - | 12 | 12 |
| T5 | - | 12 | 24 |
| T6 | 500 | - | 12 |

Trial V methodology, including animal housing and management, preparation and composition of experimental diets, as well as the organ sampling and statistical procedures were analogous to trial IV.

### Results of the in vivo validation of beneficial effects of combinations of (compound "B") with different 1,8-cineole concentrations (compound "A") on growth performance, heat shock protein response and intestinal barrier integrity in broilers under heat stress (Trial V)

Table 9 shows the analysed concentrations of Betaine, Naringin and 1,8 cineole in the diets of Trial V. The analysed values show that the minimum specifications were reached or exceeded for all experimental groups.

**Table 9: Trial V: calculated Betaine, Naringin and 1,8-cineole content of the trial diets**

| Treatment | Betaine (positive control) mg/kg feed | 1,8-cineole (compound A) mg/kg feed | Naringin (compound B) mg/kg feed | Ratio (A:B) |
|---|---|---|---|---|
| T1 | - | - | - | - |
| T2 | 500 | - | - | - |
| T3 | - | 6 | 12 | 0.5:1 |
| T4 | - | 12 | 12 | 1:1 |
| T5 | - | 18 | 12 | 1.5:1 |
| T6 | - | 24 | 12 | 2:1 |
| T7 | 500 | 12 | - | 1:0 |

Since compound "B" in the first in vivo trial showed beneficial effects on performance parameters of broilers under heat stress, which were slightly better compared to the reference substance betaine, the aim of this trial was to test a synergistic/beneficial effects of combinations of a fixed dose of compound "B" (12 mg/kg) plus increasing doses of compound "A" (6 to 24 mg/kg). The intention of this study consists in exploring combinations of compound "A" and "B" for the New Feed Additive Supporting Animal Welfare Under Heat Stress Conditions which exceed the effects of the reference substance betaine. In addition, a combination of betaine with the intermediate dose of compound "A" (12 mg/kg) was chosen as a second reference in order to test, if there exist synergistic effects of betaine and compound "A". Similarly, as in trial 1, all additives (betaine and combinations of compounds "A" and "B") unrolled already growth promoting effects in the pre- heat stress period (Figures 8 and 9). Whereas betaine alone increased daily weight gain by 2.5% compared to the negative control, the addition of 12 mg/kg compound "A" reduced this effect to nearly 1%. In contrast all combinations of compound "B" plus compound "A" exceeded the growth promoting effect of betaine alone and a fortiori that of the combination of betaine plus substance A (Figures 8 and 9). Moreover, a clear dose-response effect could be observed for the combinations "AB" (+2,1%; +2.5; +4.7%) (Figures 8 and 9). As observed in the first trial, the reference substance betaine developed a better growth promoting effect under heat stress conditions (+2.0%) compared to the negative control. Under heat stress conditions all combinations "AB" exceeded the growth performing effects of betaine alone distinctly. The dose response effect, found in the pre heat stress period, could not be observed under heat stress for the tested combinations "AB". All tested combinations "AB" increased growth performance by 5.2 to 5.6% compared to the negative control and by 3.0% to 3.5% compared to the reference substance betaine (Figures 8 and 9), respectively. Most interestingly, the combination of the reference substance betaine with the medium dose of compound"A", which failed in the pre heat stress period, unrolled in the heat stress period even slightly better growth promoting effects compared to the combinations "AB". However, since feed additives counteracting heat stress are used during the entire mast period, the combinations "AB" overall resulted in the highest improvement of final body weight compared to the negative control (+4.8%, +5.2%, +4.9%), to the reference substance betaine (+1.6%, +2.0%, +1.8%) and also compared to the combination of betaine plus the medium concentration of compound "A" (+0.2%, +0.7%, +0.4%). Very similar effects, as observed for growth performance could also be found for FCR (food conversion rate) (Figure 10).

### Trial VI: In vivo validation of beneficial effects of the final prototype combinations of 1,8-cineole with naringin (AB), or 1,8-cineole with Betalain (AC).

In the in vivo validation trial of beneficial effects of combinations of Naringin (compound "B") with different 1,8-cineole concentrations (compound "A") it turned out, that all tested combinations of "AB" were much more effective than the positive control betaine alone, and even as the combination of betaine + compound "A". Thus, consequently the aim of the last in vivo trial of the series was to test, if in addition to the combination "AB", as well as the combination "AC" unrolls stronger protection and growth performance under heat stress conditions than 500 mg/kg betaine as the positive control.

816 one-day old Ross 308 male broilers from a total of 960 were used in the experiment. Any day-old broilers, showing signs of health problems, injury, or of being too small or in poor condition were excluded in the selection process. Broilers were sexed at the hatchery. All day-old chicks were individually weighted and grouped according to weight. The birds were then assigned to the 36 pens (6 repetitions of 6 treatments), and each pen initially consisted of 34 broilers. On day 4 of the experiment in each pen, the weakest 2 broilers were removed and all pens were equated to 32 birds per pen by negative selection.

Birds of the negative control group were fed the basal diet without any further phytogenic additive. The reference group 2 was fed a diet providing 500 mg/kg betaine from a natural betaine source (Actibeet^{®}, Agrana). To the diets of groups 3 and 4 the phytogenic premixes were added providing either 12 mg Naringin/kg from citrus flavonoids, or 0,55 mg Betalain/kg from beetroot and barbary, each in combination with 12 mg 1,8-Cineole/kg from the laminaceae oil mixture (Table 10).

**Table 10: Trial VI: Experimental treatment groups**

| Treatment | Betaine (+ control) mg/kg feed | 1,8-cineole (A) mg/kg feed | Naringin (B) mg/kg feed | Betalaine (C) mg/kg feed | Ratio A:B/C |
|---|---|---|---|---|---|
| T1 | - | - | - | - | - |
| T2 | 500 | - | - | - | - |
| T3 | - | 12 | 12 | - | 1:1 |
| T4 | - | 12 | - | 55 | 0.2:1 |

Trial VI methodology, including animal housing and management, preparation and composition of experimental diets, as well as the organ sampling and statistical procedures were analogous to trial IV.

### Results of the in vivo validation of beneficial effects of the combination of the 1,8-cineole with Naringin (AB), or 1,8-cineole with Betalain (AC) on growth performance, heat shock protein response and intestinal barrier integrity in broilers under heat stress compared to betaine as the positive control (Trial VI)

In Table 11 the analysed values of the concentrations of Betaine, 1,8-Cineole, Naringin and Betalain in Trial VI are displayed.

**Table 11: Trial VI - Betaine, 1,8-cineole, Naringin and betalain content of the trial diets**

| Treatment | Betaine (+ control) mg/kg feed | 1,8-cineole (A) mg/kg feed | Naringin (B) mg/kg feed | | Betalain (C) mg/kg feed | |
|---|---|---|---|---|---|---|
| | calculated | calculated | calculated | ratio A:B | calculated | ratio A:C |
| T1 | - | - | - | - | - | - |
| T2 | 50 | - | - | - | - | - |
| T3 | - | 30 | 30 | 1:1 | - | - |
| T4 | - | 1.37 | - | - | 1.37 | 1:1 |

Due to the positive findings on additional effects of substances "B" and "C" on heat shock response in CaCo2 cells (Figure 3E) in the last trial of this series it has been evaluated, if under heat stress conditions not only combinations of substances "AB", but also combinations of substances "AC" show beneficial effects on growth performance of heat stressed broilers. In the pre-heat stress phase the positive control (betaine) and both combinations "AB" and "AC" already improved body weight by 3,60 to 4,65% compared to the untreated negative control (Figure 11). "AC" turned out as the most effective combination, whereas in this trial "AB" was slightly less effective than betaine (Figure 11). However, during the heat stress period both combinations "AB" and "AC" unrolled their high potential to improve the performance of broilers. Both additives improved final body weight by about 1%, compared to the positive control, corresponding to a 50g higher final body weight. Daily weight gain during the heat stress phase could be even improved by about 1,5% by both combinations of "AB" and "AC" compared to the positive control betaine (Figure 12). Improvement in final body weight and daily gain was also reflected by FCR (Figure 13), which was improved to a higher extent by both combinations of "AB" and "AC" compared to the positive control (betaine).

### References

Brand W, van der Wel PA, Rein MJ, Barron D, Williamson G, van Bladeren PJ, Rietjens IM. Metabolism and transport of the citrus flavonoid hesperetin in Caco-2 cell monolayers. Drug Metab Dispos. 2008 Sep;36(9):1794-802. doi: 10.1124/dmd.107.019943.
Chen RC, Sun GB, Wang J, Zhang HJ, Sun XB. Naringin protects against anoxia/reoxygenation-induced apoptosis in H9c2 cells via the Nrf2 signaling pathway. Food Funct. 2015; 6: 1331-1344.
Esatbeyoglu T, Wagner AE, Schini-Kerth VB, Rimbach G. Betanin--a food colorant with biological activity. Mol Nutr Food Res. 2015 Jan;59(1):36-47.
Ghaffarian R, Muro S. Models and methods to evaluate transport of drug delivery systems across cellular barriers. J Vis Exp. 2013 Oct 17;(80):e 50638.
Hosseini SM, Farhangfar H, Nourmohammadi R. Effects of a blend of essential oils and overcrowding stress on the growth performance, meat quality and heat shock protein gene expression of broilers. Br Poult Sci. 2018 Feb;59(1):92-99. doi: 10.1080/00071668.2017.1390209.
FangFang, Li H, Qin T, Li M, Ma S. Thymol improves high-fat diet-induced cognitive deficits in mice via ameliorating brain insulin resistance and upregulating NRF2/HO-1 pathway. Metab Brain Dis. 2017; 32: 385-393.
Kamboh AA, Hang SQ, Bakhetgul M, Zhu WY. Effects of genistein and hesperidin on biomarkers of heat stress in broilers under persistent summer stress. Poult Sci. 2013; 92: 2411-2418.
Kluth D, Banning A, Paur I, Blomhoff R, Brigelius-Flohé R. Modulation of pregnane X receptor- and electrophile responsive element-mediated gene expression by dietary polyphenolic compounds. Free Radic Biol Med. 2007 Feb 1;42(3):315-25.
König J, Wells J, Cani PD, García-Ródenas CL, MacDonald T, Mercenier A, Whyte J, Troost F, Brummer RJ. Human Intestinal Barrier Function in Health and Disease. Clin Transl Gastroenterol. 2016 Oct 20;7(10):e196. doi: 10.1038/ctg.2016.54
Lee CH, Wettasinghe M, Bolling BW, Ji LL, Parkin KL. Betalains, phase II enzyme-inducing components from red beetroot (Beta vulgaris L.) extracts. Nutr Cancer. 2005;53(1):91-103.
Maria Miguel, Antioxidants 2018; 7(4):53.
Noda S, Tanabe S, Suzuki T. Differential effects of flavonoids on barrier integrity in human intestinal Caco-2 cells. J Agric Food Chem. 2012 May 9;60(18):4628-33. doi: 10.1021/jf300382h.
Pearce SC, Mani V, Boddicker RL, Johnson JS, Weber TE, Ross JW, Rhoads RP, Baumgard LH, Gabler NK. Heat stress reduces intestinal barrier integrity and favors intestinal glucose transport in growing pigs. PLoS One. 2013 Aug 1;8(8):e70215. doi: 10.1371/journal.pone.0070215 (A).
Pearce SC, Mani V, Weber TE, Rhoads RP, Patience JF, Baumgard LH, Gabler NK. Heat stress and reduced plane of nutrition decreases intestinal integrity and function in pigs. J Anim Sci. 2013 Nov;91(11):5183-93. doi: 10.2527/jas.2013-6759 (B).
Pires Goncalves J C, de Souza Trassi M A, Lopes N B, Dörr F A, dos Santos M T, Baader W J, Olivera Jr. V X, Bastos E L. A comparative study of the purification of betanin. Food Chemistry. 2012; 131:231-238
Placha I, Takacova J, Ryzner M, Cobanova K, Laukova A, Strompfova V, Venglovska K, Faix S. Effect of thyme essential oil and selenium on intestine integrity and antioxidant status of broilers. Br Poult Sci. 2014 Feb;55(1):105-14. doi: 10.1080/00071668.20130.
Rani N, Bharti S, Manchanda M, Nag TC, Ray R, Chauhan SS, Kumari S, Arya DS. Regulation of heat shock proteins 27 and 70, p-Akt/p-eNOS and MAPKs by Naringin Dampens myocardial injury and dysfunction in vivo after ischemia/reperfusion. PLoS One. 2013 Dec 6;8(12):e82577. doi: 10.1371/journal.pone.0082577.
Saeed M, Babazadeh D, Naveed M3, Arain MA, Hassan FU, Chao S. Reconsidering betaine as a natural anti-heat stress agent in poultry industry: a review. Trop Anim Health Prod. 2017 Oct;49(7):1329-1338. doi: 10.1007/s11250-017-1355-z. Epub 2017 Jul 21.
Sharma K, Mahato N, Cho MH, Lee YR. Converting citrus wastes into value-added products: Economic and environmently friendly approaches. Nutrition. 2017 Feb;34:29-46. doi: 10.1016/j.nut.2016.09.006.
Suntar I, Khan H, Patel S, Celano R, Rastrelli L. An Overview on Citrus aurantium L.: Its Functions as Food Ingredient and Therapeutic Agent. Oxid Med Cell Longev. 2018 May 2;2018:7864269. doi: 10.1155/2018/7864269.
Türk G, Çeriba 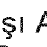 AO, 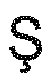 im 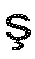 ek ÜG, Çeriba 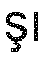 S, Güvenç M, Özer Kaya 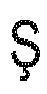 , Çiftçi M, Sönmez M, Yüce A, Bayrakdar A, Yaman M, Tonbak F. Dietary rosemary oil alleviates heat stress-induced structural and functional damage through lipid peroxidation in the testes of growing Japanese quail. Anim Reprod Sci. 2016 Jan;164:133-43. doi: 10.1016/j.anireprosci.2015.11.021.
Uritu CM, Mihai CT, Stanciu GD, Dodi G, Alexa-Stratulat T, Luca A, Leon-Constantin MM, Stefanescu R, Bild V, Melnic S, Tamba BI. Medicinal Plants of the Family Lamiaceae in Pain Therapy: A Review. Pain Res Manag. 2018 May 8;2018:7801543. doi: 10.1155/2018/7801543.
Wieten L, van der Zee R, Spiering R, Wagenaar-Hilbers J, van Kooten P, Broere F, van Eden W. A novel heat-shock protein coinducer boosts stress protein Hsp70 to activate T cell regulation of inflammation in autoimmune arthritis. Arthritis Rheum. 2010 Apr;62(4):1026-35. doi: 10.1002/art.27344.
Zhu C, Dong Y, Liu H, Ren H, Cui Z. Hesperetin protects against H2O2-triggered oxidative damage via upregulation of the Keap1-Nrf2/HO-1 signal pathway in ARPE-19 cells. Biomed Pharmacother. 2017; 88:124-133.
Zou Y, Wang J, Peng J, Wei H. Oregano Essential Oil Induces SOD1 and GSH Expression through Nrf2 Activation and Alleviates Hydrogen Peroxide-Induced Oxidative Damage in IPEC-J2 Cells. Oxid Med Cell Longev. 2016;2016:5987183. doi: 10.1155/2016/5987183 (A).
Zou Y, Xiang Q, Wang J, Peng J, Wei H. Oregano Essential Oil Improves Intestinal Morphology and Expression of Tight Junction Proteins Associated with Modulation of Selected Intestinal Bacteria and Immune Status in a Pig Model. Biomed Res Int. 2016;2016:5436738. doi: 10.1155/2016/5436738 (B).

### The invention will now be defined by reference to the following clauses

1. A livestock feed additive comprising at least 1,5% 1,8 cineole (w/w) and at least one of naringin or betalain.
2. The feed additive of clause 1, which comprises up to 6% 1,8 cineole (w/w).
3. The feed additive of clause 1 or 2, which comprises at least 3% naringin (w/w), preferably up to 6% (w/w), and/or at least 0,1% betalain (w/w), preferably up to 3% (w/w).
4. The feed additive of any of clauses 1 to 3, which comprises
   a) 1,8 cineole and naringin at a ratio of at least 0.2:1 (w/w); and/or
   b) 1,8 cineole and betalain at a ratio of at least 4:1 (w/w).
5. The feed additive of any of clauses 1 to 4, wherein said 1,8 cineole is comprised in an essential oil, preferably of lamiaceae plants, preferably of *rosemarinus genus.*
6. The feed additive of any of clauses 1 to 5, wherein said naringin is comprised in citrus plant material, preferably of orange or grapefruit.
7. The feed additive of any of clauses 1 to 6, wherein said betalain is comprised in plant material of plants of the Caryophyllales order, preferably of the amaranthaceae family, preferably of beta vulgaris.
8. The feed additive of any of clauses 1 to 7, which comprises a flowable mixture of phytogenic compounds, preferably comprising one or more of essential oils, dried herbs, spices, carbohydrates, bulking or anti-caking agents, or further excipients.
9. The feed additive of any of clauses 1 to 8, which further comprises at least one essential oil comprising carvacrol and/or thymol.
10. The feed additive of clause 9, which comprises at least 0,05% carvacrol (w/w) and/or at least 0,05% thymol (w/w).
11. The feed additive of any of clauses 1 to 10, which is a storage stable, pelletable dry preparation, with a stability of at least 18 months at room temperature.
12. The feed additive of any of clauses 1 to 11, which is provided in a preparation for use in preparing a feed product for poultry, swine or ruminants.
13. A feed product comprising the feed additive composition of any of clauses 1 to 12, preferably wherein the feed product comprises
   a) 1,8 cineole at a concentration of at least 5 mg/kg; and
   b) naringin at a concentration of at least 10 mg/kg and/or betalain at a concentration of at least 0,3 mg/kg.
14. Use of the feed additive of any of clauses 1 to 12 or the feed product of clause 13, for breeding livestock at risk of heat stress.
15. A method of breeding livestock at risk of heat stress comprising feeding the feed product of clause 13.

## Claims

1. A livestock feed additive for use in treating livestock at risk of or suffering from heat stress during breeding, the livestock feed additive comprising 1,8-cineole (w/w) and at least one of naringin or betalain.

2. The feed additive for the use of claim 1, wherein the feed additive comprises at least 1.5% 1,8-cineole (w/w).

3. The feed additive for the use according to claim 1 or 2, comprising up to 6% 1,8-cineole (w/w).

4. The feed additive for the use according to any one of claims 1-3, comprising at least 3% naringin (w/w), preferably up to 6% (w/w), and/or at least 0.1 % betalain (w/w), preferably up to 3% (w/w).

5. The feed additive for the use according to any of claims 1 to 4, comprising:
a) 1,8-cineole and naringin at a ratio of at least 0.2:1 (w/w); and/or
b) 1,8-cineole and betalain at a ratio of at least 4:1 (w/w).

6. The feed additive for the use according to any of claims 1 to 5, wherein said 1,8-cineole is comprised in an essential oil, preferably of lamiaceae plants, preferably of *rosemarinus genus.*

7. The feed additive for the use according to any of claims 1 to 6, wherein said naringin is comprised in citrus plant material, preferably of orange or grapefruit.

8. The feed additive for the use according to any of claims 1 to 7, wherein said betalain is comprised in plant material of plants of the Caryophyllales order, preferably of the amaranthaceae family, preferably of beta vulgaris.

9. The feed additive for the use according to any of claims 1 to 8, which comprises a flowable mixture of phytogenic compounds, preferably comprising one or more of essential oils, dried herbs, spices, carbohydrates, bulking or anti-caking agents, or further excipients.

10. The feed additive for the use according to any of claims 1 to 9, which further comprises at least one essential oil comprising carvacrol and/or thymol.

11. The feed additive for the use according to claim 10, which comprises at least 0.05% carvacrol (w/w) and/or at least 0.05% thymol (w/w).

12. The feed additive for the use according to any of claims 1 to 11, which is a storage stable, pelletable dry preparation, with a stability of at least 18 months at room temperature.

13. The feed additive for the use according to any of claims 1 to 12, wherein the livestock is poultry, swine or ruminants.
